# EUROPEAN PATENT SPECIFICATION

(11) **EP 2 756 084 B1**
(45) Date of publication and mention of the grant of the patent: **03.06.2020**
(21) Application number: 12831494.5
(22) Date of filing: 12.09.2012
(51) Int. Cl.: A01H 3/04, A01N 43/80, C07H 21/04, C12N 9/10, C12N 9/88, C12N 15/82

(54) **METHODS AND COMPOSITIONS FOR WEED CONTROL**
VERFAHREN UND ZUSAMMENSETZUNGEN ZUR UNKRAUTBEKÄMPFUNG
PROCÉDÉS ET COMPOSITIONS DE LUTTE CONTRE LES MAUVAISES HERBES

(30) Priority: 13.09.2011 US 201161534061 P
(43) Date of publication of application: 23.07.2014
(73) Proprietor: Monsanto Technology LLC, St. Louis, MO 63167 (US)
(72) Inventor: ADER, Daniel, St. Louis, MO 63167 (US); FINNESSY, John, J., St. Louis, MO 63167 (US); LI, Zhaolong, St. Louis, MO 63167 (US); SHAH, Ronak, Hasmukh, St. Louis, MO 63167 (US); TAO, Nengbing, St. Louis, MO 63167 (US); TAYLOR, Christina, Marie, St. Louis, MO 63167 (US); TAYLOR, Jennifer, Chou, St. Louis, MO 63167 (US); WANG, Dafu, St. Louis, MO 63167 (US); WEAVER, Lisa, Marie, St. Louis, MO 63167 (US)
(74) Representative: Uexküll & Stolberg
(86) International application number: PCT/US2012/054814
(87) International publication number: WO 2013/040005

(56) References cited:
- WO-A1-2011/112570
- WO-A2-2004/062351
- US-A1- 2006 009 358
- US-A1- 2006 130 172
- RAINER HOFGEN ' ET AL: "Repression of Acetolactate Synthase Activity through Antisense lnhibition Molecular and Biochemical Analysis of Transgenic Potato (Solanum fuberosum L. cv DCsirCe) Plants", PLANT PHYSIOL, vol. 107, 1 January 1995 (1995-01-01), pages 469-477, XP055146754,
- ANDERSSON M ET AL: "A novel selection system for potato transformation using a mutated AHAS gene.", PLANT CELL REPORTS, vol. 22, no. 4, November 2003 (2003-11), pages 261-267, XP002735721, ISSN: 0721-7714
- OUELLET T ET AL: "MEMBERS OF THE ACETOHYDROXYACID SYNTHASE MULTIGENE FAMILY OF BRASSICA NAPUS HAVE DIVERGENT PATTERNS OF EXPRESSION", THE PLANT JOURNAL, BLACKWELL SCIENTIFIC PUBLICATIONS, OXFORD, GB, vol. 2, no. 3, 1 January 1992 (1992-01-01) , pages 321-330, XP002008881, ISSN: 0960-7412
- GAO DONGLI ET AL: "Down-regulation of acetolactate synthase compromises Ol-1-mediated resistance to powdery mildew in tomato", BMC PLANT BIOLOGY, vol. 14, January 2014 (2014-01), XP002735722,
- HOFGEN ET AL.: 'Repression of Acetolactate Synthase Activity through Antisense Inhibition 0 (Molecular and Biochemical Analysis of Transgenic Potato (Solanum tuberosum L. cv Desiree) Plants).' PLANT PHYSIOL vol. 107, no. 2, February 1995, pages 469 - 477, XP055146754
- ZHANG ET AL.: 'Agrobacterium-mediated transformation of Arabidopsis thaliana using the floral dip method.' NATURE PROTOCOLS vol. 1, no. 2, 2006, pages 1 - 6, XP055146758
- VENCILL ET AL.: 'Resistance of Weeds to Herbicides.' HERBICIDES AND ENVIRONMENT. 08 January 2011, NEW YORK, pages 585 - 594, XP055146760 ISBN: 978-953-307-4 Retrieved from the Internet: <URL:http://www.intechopen.com/books/herbic ides-and-environment/resistance-of-weeds -to- herbicides>
- DATABASE GENBANK [Online] 05 August 2007 'Amaranthus hypochondriacus acetolactate synthase (ALS) gene, complete cds', XP003032592 Database accession no. EU024568

## Description

This application claims benefit under 35USC § 119(e) of United States provisional application serial no. 61/534,061 filed 09/13/2011

. The sequence listing that is contained in the file named "40_21(58635)B seq listing.txt", which is 1,997,877 bytes (measured in operating system MS-Windows) and was created on 9 Sept 2012.

### FIELD

The invention relates generally to the field of weed management. More specifically, the invention relates to acetolactate synthase genes in weedy plants and compositions containing polynucleotide molecules for modulating their expression. The invention further provides methods and compositions useful for weed control.

### BACKGROUND

Weeds are plants that compete with cultivated plants in an agronomic environment and cost farmers billions of dollars annually in crop losses and the expense of efforts to keep weeds under control. Weeds also serve as hosts for crop diseases and insect pests. The losses caused by weeds in agricultural production environments include decreases in crop yield, reduced crop quality, increased irrigation costs, increased harvesting costs, reduced land value, injury to livestock, and crop damage from insects and diseases harbored by the weeds. The principal means by which weeds cause these effects are: 1) competing with crop plants for water, nutrients, sunlight and other essentials for growth and development, 2) production of toxic or irritant chemicals that cause human or animal health problem, 3) production of immense quantities of seed or vegetative reproductive parts or both that contaminate agricultural products and perpetuate the species in agricultural lands, and 4) production on agricultural and nonagricultural lands of vast amounts of vegetation that must be disposed of. Herbicide tolerant weeds are a problem with nearly all herbicides in use, there is a need to effectively manage these weeds. There are over 365 weed biotypes currently identified as being herbicide resistant to one or more herbicides by the Herbicide Resistance Action Committee (HRAC), the North American Herbicide Resistance Action Committee (NAHRAC), and the Weed Science Society of America (WSSA).

The ALS (acetolactate synthase, also known as acetohydroxyacid synthase, AHAS) enzyme catalyzes the first step in the synthesis of the branched-chain amino acids (valine, leucine, and isoleucine). ALS includes a large subunit member and a small subunit member that function to provide regulated production of the branched-chain amino acids. This enzyme is the target of many ALS inhibiting herbicides that include members of the chemical families of Sulfonylureas, Imidazolinones, Triazolopyrimidines, Pyrimidinyl(thio)benzoates, and Sulfonylaminocarbonyl-triazolinones. Studies have shown that the ALS repression via antisense expression of the ALS gene results in severe growth retardation and strong phenotypical effects in potato plants, which resembles the effects caused by ALS-inhibiting herbicides (Rainer Hofgen et al, Plant Physiol, 1995).

### SUMMARY

In one aspect, the invention provides a method of plant control comprising: topically applying to a surface of a plant a composition comprising a double-stranded RNA (dsRNA) polynucleotide and a transfer agent, wherein said dsRNA polynucleotide is identical or complementary to at least 18 contiguous nucleotides of an RNA sequence of an acetolactate synthase (ALS) gene sequence, wherein said ALS gene sequence is selected from the group consisting of SEQ ID NOs:2, 15, 25-27, 30-33, 35, 36, 39-45, 1692-1697, 1699, 1705, 1707, 1709-1712, 1714-1721, 1727-1742, 1744-1774, and 1776-1788, wherein said transfer agent conditions said surface of said plant for permeation by said dsRNA polynucleotide, and whereby said plant's growth, development, or reproductive ability is reduced or said plant is more sensitive to an ALS inhibitor herbicide, relative to an untreated plant.

In a further embodiment, the transfer agent is an organosilicone surfactant composition or an organosilicone compound contained therein; the plant is selected from the group consisting of *Amaranthus palmeri, Amaranthus rudis, Amaranthus chlorostachys, Amaranthus graecizans, Amaranthus hybridus, Amaranthus lividus, Amaranthus spinosus, Amaranthus thunbergii, Amaranthus viridis, Ambrosia trifida, Kochia scoparia, Abutilon theophrasti, Chenopodium album, Commelina diffusa, Conyza canadensis Digitaria sanguinalis, Euphorbia heterophylla,* and *Lolium multiflorum;* or the composition further comprises : (a) said ALS inhibitor herbicide; or (b) one or more herbicides different from said ALS inhibitor herbicide.

In another embodiment, the dsRNA polynucleotide is at least 19 contiguous nucleotides in length, or the composition comprises any combination of two or more of said dsRNA polynucleotides.

In another aspect, the invention provides a composition for topical application to a surface of a plant comprising a dsRNA polynucleotide and a transfer agent, wherein said dsRNA polynucleotide is identical or complementary to at least 18 contiguous nucleotides of an RNA sequence of an ALS gene sequence, wherein said ALS gene sequence is selected from the group consisting of SEQ ID NOs:2, 15, 25-27, 30-33, 35, 36, 39-45, 1692-1697, 1699, 1705, 1707, 1709-1712, 1714-1721, 1727-1742, 1744-1774, and 1776-1788, wherein said transfer agent conditions said surface of said plant for permeation by said dsRNA polynucleotide, and whereby said plant treated with said composition has its growth, development, or reproductive ability suppressed or delayed or said plant is more sensitive to an ALS inhibitor herbicide as a result of said composition comprising said dsRNA polynucleotide, relative to an untreated plant.

In some embodiment, the transfer agent is an organosilicone preparation; the dsRNA polynucleotide is at least 19 contiguous nucleotides in length; the dsRNA polynucleotide is selected from the group consisting of SEQ ID NOs:48-53, 62, 63, 66-73, 76, 77, 84, 85, 90, 91, 94-97, 100, 101, 110, 111, 114-117, 120, 121, 124, 125, 138, 139, 142-145, 148-171, 186-189, 192, 193, 196-239, 250, 251, 254-256, 257, 260, 261, 264-279, 288, 289, 294, 295, 306, 307, 310-339, 352-359, 362-365, 378-385, 388-393, 396-419, 428, 429, 432, 433, 436, 437, 440, 441, 450, 451, 454-457, 460, 461, 464-467, 476, 477, 480, 481, 486, 487, 490-493, 502, 503, 506-509, 512, 513, 516-519, 528, 529, 532-535, 538, 539, 542-559, 564-581, 584-609, 612-631, 634-637, 640-659, 662-665, 668-919, 922-1363, 1789-1840, 1843-1916, 1925-2024, 2027-2040, 2043-2060, 2063-2102, 2105-2270, 2273-2286, 2289-2404, 2407-2640, 2647-2662, 2665-2678, 2681-2696, 2699-2956, 2959-3946, and 3949-4166; the dsRNA polynucleotide is selected from the group consisting of SEQ ID NOs:1364-1691 and 4167-4201; or the composition further comprises (a) said ALS inhibitor herbicide;(b) said ALS inhibitor molecule selected from the group consisting of amidosulfuron, azimsulfuron, bensulfuron-methyl, chlorimuron-ethyl, chlorsulfuron, cinosulfuron, cyclosulfamuron, ethametsulfuron-methyl, ethoxysulfuron, flazasulfuron, flupyrsulfuron-methyl-Na, foramsulfuron, halosulfuron-methyl, imazosulfuron, iodosulfuron, metsulfuron-methyl, nicosulfuron, oxasulfuron, primisulfuron-methyl, prosulfuron, pyrazosulfuron-ethyl, rimsulfuron, sulfometuron-methyl, sulfosulfuron, thifensulfuron-methyl, triasulfuron, tribenuron-methyl, trifloxysulfuron, triflusulfuron-methyl, tritosulfuron, imazapic, imazamethabenz-methyl, imazamox, imazapyr, imazaquin, imazethapyr, cloransulam-methyl, diclosulam, florasulam, flumetsulam, metosulam, bispyribac-Na, pyribenzoxim, pyriftalid, pyrithiobac-Na, pyriminobac-methyl, flucarbazone-Na, and procarbazone-Na; or (c) a co-herbicide.

In another aspect, the invention provides a method of reducing expression of an ALS gene in a plant comprising: topically applying to a surface of said plant a composition comprising a dsRNA polynucleotide and a transfer agent, wherein said dsRNA polynucleotide is identical or complementary to at least 18 contiguous nucleotides of an RNA sequence of an ALS gene sequence, wherein said ALS gene sequence is selected from the group consisting of SEQ ID NOs:2, 15, 25-27, 30-33, 35, 36, 39-45, 1692-1697, 1699, 1705, 1707, 1709-1712, 1714-1721, 1727-1742, 1744-1774, and 1776-1788, wherein said transfer agent conditions said surface of said plant for permeation by said dsRNA polynucleotide, whereby said expression of said ALS gene is reduced, relative to an untreated plant. In some embodiments, the transfer agent is an organosilicone preparation, or the dsRNA polynucleotide is at least 19 contiguous nucleotides in length.

In yet another aspect, the invention provides a method of identifying dsRNA polynucleotides useful in modulating ALS gene expression when topically applied to a surface of a plant comprising: a) providing a plurality of dsRNA polynucleotides that comprise a region identical or complementary to at least 18 contiguous nucleotides of an RNA sequence of an ALS gene sequence selected from the group consisting of SEQ ID NOs:2, 15, 25-27, 30-33, 35, 36, 39-45, 1692-1697, 1699, 1705, 1707, 1709-1712, 1714-1721, 1727-1742, 1744-1774, and 1776-1788; b) topically applying to said surface of said plant a composition comprising one or more of said dsRNA polynucleotides and a transfer agent; and c) analyzing said plant, or a plant extract thereof, for modulation of ALS gene expression, wherein said transfer agent conditions said surface of said plant for permeation by said one or more of said dsRNA polynucleotides, and whereby said plant treated with said composition has its growth, development, or reproductive ability suppressed or delayed or said plant is more sensitive to an ALS inhibitor herbicide as a result of said composition, relative to an untreated plant.

In a further embodiment, the plant is selected from the group consisting of *Amaranthus palmeri, Amaranthus rudis, Amaranthus chlorostachys, Amaranthus graecizans, Amaranthus hybridus, Amaranthus lividus, Amaranthus spinosus, Amaranthus thunbergii, Amaranthus viridis, Ambrosia trifida, Kochia scoparia, Abutilon theophrasti, Chenopodium album, Commelina diffusa, Conyza canadensis Digitaria sanguinalis, Euphorbia heterophylla,* and *Lolium multiflorum;* or the ALS gene expression is reduced relative to a plant not treated with said composition; or the transfer agent is an organosilicone preparation.

In still another aspect, the invention provides an agricultural chemical composition for topical application to a surface of a plant comprising an admixture of a dsRNA polynucleotide, glyphosate, and a co-herbicide, wherein said dsRNA polynucleotide is identical or complementary to at least 18 contiguous nucleotides of an RNA sequence of an ALS gene sequence, wherein said ALS gene sequence is selected from the group consisting of SEQ ID NOs:2, 15, 25-27, 30-33, 35, 36, 39-45, 1692-1697, 1699, 1705, 1707, 1709-1712, 1714-1721, 1727-1742, 1744-1774, and 1776-1788, and whereby said plant treated with said composition has its growth, development, or reproductive ability suppressed or delayed or said plant is more sensitive to an ALS inhibitor herbicide as a result of said composition comprising said dsRNA polynucleotide, relative to an untreated plant. In some embodiments, the co-herbicide is selected from the group consisting of amide herbicides, arsenical herbicides, benzothiazole herbicides, benzoylcyclohexanedione herbicides, benzofuranyl alkylsulfonate herbicides, carbamate herbicides, cyclohexene oxime herbicides, cyclopropylisoxazole herbicides, dicarboximide herbicides, dinitroaniline herbicides, dinitrophenol herbicides, diphenyl ether herbicides, dithiocarbamate herbicides, halogenated aliphatic herbicides, imidazolinone herbicides, inorganic herbicides, nitrile herbicides, organophosphorus herbicides, oxadiazolone herbicides, oxazole herbicides, phenoxy herbicides, phenylenediamine herbicides, pyrazole herbicides, pyridazine herbicides, pyridazinone herbicides, pyridine herbicides, pyrimidinediamine herbicides, pyrimidinyloxybenzylamine herbicides, quaternary ammonium herbicides, thiocarbamate herbicides, thiocarbonate herbicides, thiourea herbicides, triazine herbicides, triazinone herbicides, triazole herbicides, triazolone herbicides, triazolopyrimidine herbicides, uracil herbicides, and urea herbicides.

In one aspect, the invention provides an agricultural chemical composition for topical application to a surface of a plant comprising an admixture of a dsRNA polynucleotide, glyphosate, and a pesticide, wherein said dsRNA polynucleotide is identical or complementary to at least 18 contiguous nucleotides of an RNA sequence of an ALS gene sequence, wherein said ALS gene sequence is selected from the group consisting of SEQ ID NOs:2, 15, 25-27, 30-33, 35, 36, 39-45, 1692-1697, 1699, 1705, 1707, 1709-1712, 1714-1721, 1727-1742, 1744-1774, and 1776-1788, and whereby said plant treated with said composition has its growth, development, or reproductive ability suppressed or delayed or said plant is more sensitive to an ALS inhibitor herbicide as a result of said composition comprising said dsRNA polynucleotide, relative to an untreated plant. In some embodiments, the pesticide is selected from the group consisting of insecticides, fungicides, nematicides, bactericides, acaricides, growth regulators, chemosterilants, semiochemicals, repellents, attractants, pheromones, feeding stimulants, and biopesticides.

In another aspect, the invention provides a dsRNA polynucleotide for topical application to a surface of a plant, wherein said dsRNA polynucleotide comprises a polynucleotide identical or complementary to at least 18 contiguous nucleotides of an RNA sequence of a polynucleotide selected from the group consisting of SEQ ID NOs:4229-4235, whereby said plant treated with said dsRNA polynucleotide has its sensitivity to a glyphosate containing herbicide composition enhanced, relative to untreated plant.

### BRIEF DESCRIPTION OF THE FIGURES

The following drawings form part of the present specification and are included to further demonstrate certain aspects of the present invention. The invention may be better understood by reference to one or more of these drawings in combination with the detailed description of specific embodiments presented herein. The invention can be more fully understood from the following description of the figures:
Figure 1. Treatment of Amaranthus palmer plants with ssDNA trigger polynucleotides and ALS inhibitor herbicide (Staple®).
Figure 2. Treatment of Amaranthus palmer plants with the ALS_pro_S1 trigger molecule demonstrating improved activity of the Staple® and Classic® herbicides.

### DETAILED DESCRIPTION

The invention provides a method and compositions containing a polynucleotide that provide for regulation of an ALS (acetolactate synthase) large subunit and ALS small subunit gene expression and enhanced control of weedy plant species amd importantly ALS inhibitor resistant weed biotypes. Aspects of the method can be applied to manage various weedy plants in agronomic and other cultivated environments.

The following definitions and methods are provided to better define the present invention and to guide those of ordinary skill in the art in the practice of the present invention. Unless otherwise noted, terms are to be understood according to conventional usage by those of ordinary skill in the relevant art. Where a term is provided in the singular, the inventors also contemplate aspects of the invention described by the plural of that term.

By "non-transcribable" polynucleotides is meant that the polynucleotides do not comprise a complete polymerase II transcription unit.

As used herein "solution" refers to homogeneous mixtures and non-homogeneous mixtures such as suspensions, colloids, micelles, and emulsions.

Weedy plants are plants that compete with cultivated plants, those of particular importance include, but are not limited to important invasive and noxious weeds in crop production, such as, *Amaranthus* species *-A. albus, A. blitoides, A. hybridus, A. palmeri, A. powellii, A. retroflexus, A. spinosus, A. tuberculatus,* and *A. viridis; Ambrosia* species - *A. trifida, A. artemisifolia; Lolium* species *-L. multiflorum, L. rigidium, L perenne; Digitaria* species *-D. insularis; Euphorbia* species *-E. heterophylla; Kochia species* - *K. scoparia; Sorghum* species *-S. halepense; Conyza* species -*C*. *bonariensis, C. canadensis, C. sumatrensis; Chloris* species -*C*. *truncate; Echinochola* species - *E. colona, E. crus-galli; Eleusine species -E. indica; Poa* species *-P. annua; Plantago* species *-P. lanceolata; Avena species* - *A. fatua; Chenopodium* species - *C. album; Setaria* species - *S. viridis, Abutilon theophrasti, Ipomoea* species, *Sesbania,* species, *Cassia* species, *Sida* species, *Brachiaria,* species and *Solanum* species.

Additional weedy plant species found in cultivated areas include *Alopecurus myosuroides, Avena sterilis, Avena sterilis ludoviciana, Brachiaria plantaginea, Bromus diandrus, Bromus rigidus, Cynosurus echinatus, Digitaria ciliaris, Digitaria ischaemum, Digitaria sanguinalis, Echinochloa oryzicola, Echinochloa phyllopogon, Eriochloa punctata, Hordeum glaucum, Hordeum leporinum, Ischaemum rugosum, Leptochloa chinensis, Lolium persicum,*, *Phalaris minor, Phalaris paradoxa, Rottboellia exalta, Setaria faberi, Setaria viridis var, robusta-alba schreiber, Setaria viridis var, robusta -purpurea, Snowdenia polystachea, Sorghum sudanese, Alisma plantago-aquatica, Amaranthus lividus, Amaranthus quitensis, Ammania auriculata, Ammania coccinea, Anthemis cotula, Apera spica-venti, Bacopa rotundifolia, Bidens pilosa, Bidens subalternans, Brassica tournefortii, Bromus tectorum, Camelina microcarpa, Chrysanthemum coronarium, Cuscuta campestris, Cyperus difformis, Damasonium minus, Descurainia sophia, Diplotaxis tenuifolia, Echium plantagineum, Elatine triandra var, pedicellata, Euphorbia heterophylla, Fallopia convolvulus, Fimbristylis miliacea, Galeopsis tetrahit, Galium spurium, Helianthus annuus, Iva xanthifolia, Ixophorus unisetus, Ipomoea indica, Ipomoea purpurea, Ipomoea sepiaria, Ipomoea aquatic, Ipomoea triloba, Lactuca serriola, Limnocharis flava, Limnophila erecta, Limnophila sessiliflora, Lindernia dubia, Lindernia dubia var, major, Lindernia micrantha, Lindernia procumbens, Mesembryanthemum crystallinum, Monochoria korsakowii, Monochoria vaginalis, Neslia paniculata, Papaver rhoeas, Parthenium hysterophorus, Pentzia suffruticosa, Phalaris minor, Raphanus raphanistrum, Raphanus sativus, Rapistrum rugosum, Rotala indica var, uliginosa, Sagittaria guyanensis, Sagittaria montevidensis, Sagittaria pygmaea, Salsola iberica, Scirpus juncoides var, ohwianus, Scirpus mucronatus, Setaria lutescens, Sida spinosa, Sinapis arvensis, Sisymbrium orientale, Sisymbrium thellungii, Solanum ptycanthum, Sonchus asper, Sonchus oleraceus, Sorghum bicolor, Stellaria media, Thlaspi arvense, Xanthium strumarium, Arctotheca calendula, Conyza sumatrensis, Crassocephalum crepidiodes, Cuphea carthagenenis, Epilobium adenocaulon, Erigeron philadelphicus, Landoltia punctata, Lepidium virginicum, Monochoria korsakowii, Solanum americanum, Solanum nigrum, Vulpia bromoides, Youngia japonica, Hydrilla verticillata, Carduus nutans, Carduus pycnocephalus, Centaurea solstitialis, Cirsium arvense, Commelina diffusa, Convolvulus arvensis, Daucus carota, Digitaria ischaemum, Echinochloa crus-pavonis, Fimbristylis miliacea, Galeopsis tetrahit, Galium spurium, Limnophila erecta, Matricaria perforate, Papaver rhoeas, Ranunculus acris, Soliva sessilis, Sphenoclea zeylanica, Stellaria media, Nassella trichotoma, Stipa neesiana, Agrostis stolonifera, Polygonum aviculare, Alopecurus japonicus, Beckmannia syzigachne, Bromus tectorum, Chloris inflate, Echinochloa erecta, Portulaca oleracea, and Senecio vulgaris.* It is believed that all plants contain a acetolactate synthase gene in their genome, the sequence of which can be isolated and polynucleotides made according to the methods of the present invention that are useful for regulation, suppressing or delaying the expression of the target ALS gene (either the large subunit or the small subunit or both) in the plants and the growth or development of the treated plants.

A cultivated plant may also be weedy plant when it occurs in unwanted environments. For example, corn plants growing in a soybean field. Transgenic crops with one or more herbicide tolerances will need specialized methods of management to control weeds and volunteer crop plants. The present invention enables the targeting of a transgene for herbicide tolerance to permit the treated plants to become sensitive to the herbicide. For example, transgene ALS DNA sequences in transgenic events that include DP-356043-5.

A "trigger" or "trigger polynucleotide" is a polynucleotide molecule that is homologous or complementary to a target gene polynucleotide. The trigger polynucleotide molecules modulate expression of the target gene when topically applied to a plant surface with a transfer agent, whereby a plant treated with said composition has its growth or development or reproductive ability regulated, suppressed or delayed or said plant is more sensitive to an ALS inhibitor herbicide as a result of said polynucleotide containing composition relative to a plant not treated with a composition containing the trigger molecule. Trigger polynucleotides disclosed herein are generally described in relation to the target gene sequence and maybe used in the sense (homologous) or antisense (complementary) orientation as single stranded molecules or comprise both strands as double stranded molecules or nucleotide variants and modified nucleotides thereof depending on the various regions of a gene being targeted.

It is contemplated that the composition of the present invention will contain multiple polynucleotides and herbicides that include but not limited to ALS gene trigger polynucleotides and an ALS inhibitor herbicide and anyone or more additional herbicide target gene trigger polynucleotides and the related herbicides and anyone or more additional essential gene trigger polynucleotides. Essential genes are genes in a plant that provide key enzymes or other proteins, for example, a biosynthetic enzyme, metabolizing enzyme, receptor, signal transduction protein, structural gene product, transcription factor, or transport protein; or regulating RNAs, such as, microRNAs, that are essential to the growth or survival of the organism or cell or involved in the normal growth and development of the plant (Meinke, et al., Trends Plant Sci. 2008 Sep;13(9):483-91). The suppression of an essential gene enhances the effect of a herbicide that affects the function of a gene product different than the suppressed essential gene. The compositions of the present invention can include various trigger polynucleotides that modulate the expression of an essential gene other than ALS.

Herbicides for which transgenes for plant tolerance have been demonstrated and the method of the present invention can be applied, include but are not limited to: auxin-like herbicides, glyphosate, glufosinate, sulfonylureas, imidazolinones, bromoxynil, delapon, dicamba, cyclohezanedione, protoporphyrionogen oxidase inhibitors, 4-hydroxyphenyl-pyruvate-dioxygenase inhibitors herbicides. For example, transgenes and their polynucleotide molecules that encode proteins involved in herbicide tolerance are known in the art, and include, but are not limited to an 5-enolpyruvylshikimate-3-phosphate synthase (EPSPS), for example, as more fully described in U.S. Pat. Nos. 7,807,791 (SEQ ID NO:5); 6,248,876 B1; 5,627,061; 5,804,425; 5,633,435; 5,145,783; 4,971,908; 5,312,910; 5,188,642; 4,940,835; 5,866,775; 6,225,114 B1; 6,130,366; 5,310,667; 4,535,060; 4,769,061; 5,633,448; 5,510,471; U.S. Pat. No. Re. 36,449; U.S. Pat. Nos. RE 37,287 E; and 5,491,288; tolerance to sulfonylurea and/or imidazolinone, for example, as described more fully in U.S. Pat. Nos. 5,605,011; 5,013,659; 5,141,870; 5,767,361; 5,731,180; 5,304,732; 4,761,373; 5,331,107; 5,928,937; and 5,378,824; and international publication WO 96/33270; tolerance to hydroxyphenylpyruvatedioxygenases inhibitiong herbicides in plants are described in U.S. Pat. Nos. 6,245,968 B1; 6,268,549; and 6,069,115; and US7,312,379 SEQ ID NO:3; US7,935,869; US7,304,209, SEQ ID NO:1, 3,5 and 15; aryloxyalkanoate dioxygenase polynucleotides, which confer tolerance to 2,4-D and other phenoxy auxin herbicides as well as to aryloxyphenoxypropionate herbicides as described, for example, in WO2005/107437; US7,838,733 SEQ ID NO:5;) and dicamba-tolerance polynucleotides as described, for example, in Herman et al. (2005) J. Biol. Chem. 280: 24759-24767. Other examples of herbicide-tolerance traits include those conferred by polynucleotides encoding an exogenous phosphinothricin acetyltransferase, as described in U.S. Pat. Nos. 5,969,213; 5,489,520; 5,550,318; 5,874,265; 5,919,675; 5,561,236; 5,648,477; 5,646,024; 6,177,616; and 5,879,903. Plants containing an exogenous phosphinothricin acetyltransferase can exhibit improved tolerance to glufosinate herbicides, which inhibit the enzyme glutamine synthase. Additionally, herbicide-tolerance polynucleotides include those conferred by polynucleotides conferring altered protoporphyrinogen oxidase (protox) activity, as described in U.S. Pat. Nos. 6,288,306 B1; 6,282,837 B1; and 5,767,373; and WO 01/12825. Plants containing such polynucleotides can exhibit improved tolerance to any of a variety of herbicides which target the protox enzyme (also referred to as protox inhibitors). Polynucleotides encoding a glyphosate oxidoreductase and a glyphosate-N-acetyl transferase (GOX described in U.S. Patent 5,463,175 and GAT described in U.S. Patent publication 20030083480, dicamba monooxygenase U.S. Patent publication 20030135879
; a polynucleotide molecule encoding bromoxynil nitrilase (*Bxn* described in U.S. Patent No. 4,810,648 for Bromoxynil tolerance
; a polynucleotide molecule encoding phytoene desaturase (*crtI*) described in Misawa et al, (1993) Plant J. 4:833-840 and Misawa et al, (1994) Plant J. 6:481-489 for norflurazon tolerance; a polynucleotide molecule encoding acetohydroxyacid synthase (AHAS, *aka* ALS) described in Sathasiivan et al. (1990) Nucl. Acids Res. 18:2188-2193 for tolerance to sulfonylurea herbicides; and the *bar* gene described in DeBlock, et al. (1987) EMBO J. 6:2513-2519 for glufosinate and bialaphos tolerance. The transgenic coding regions and regulatory elements of the herbicide tolerance genes are targets in which polynucleotide triggers and herbicides can be included in the composition of the present invention.

The composition of the present invention include a component that is an ALS inhibitor herbicide which includes but are not limited to amidosulfuron, azimsulfuron, bensulfuron-methyl, chlorimuron-ethyl, chlorsulfuron ,cinosulfuron, cyclosulfamuron, ethametsulfuron-methyl, ethoxysulfuron, flazasulfuron, flupyrsulfuron-methyl-Na, foramsulfuron, halosulfuron-methyl, imazosulfuron, iodosulfuron, metsulfuron-methyl ,nicosulfuron ,oxasulfuron ,primisulfuron-methyl, prosulfuron, pyrazosulfuron-ethyl, rimsulfuron, sulfometuron-methyl, sulfosulfuron, thifensulfuron-methyl, triasulfuron, tribenuron-methyl, trifloxysulfuron, triflusulfuron-methyl, tritosulfuron, imazapic, imazamethabenz-methyl, imazamox, imazapyr, imazaquin, imazethapyr, cloransulam-methyl, diclosulam, florasulam, flumetsulam, metosulam, bispyribac-Na, pyribenzoxim, pyriftalid, pyrithiobac-Na, pyriminobac-methyl, flucarbazone-Na, and procarbazone-Na.

Numerous herbicides (herein refered to as co-herbicides) are available that can be added to the composition, for example, members of the herbicide families that include but are not limited to amide herbicides, aromatic acid herbicides, arsenical herbicides, benzothiazole herbicides, benzoylcyclohexanedione herbicides, benzofuranyl alkylsulfonate herbicides, carbamate herbicides, cyclohexene oxime herbicides, cyclopropylisoxazole herbicides, dicarboximide herbicides, dinitroaniline herbicides, dinitrophenol herbicides, diphenyl ether herbicides, dithiocarbamate herbicides, halogenated aliphatic herbicides, imidazolinone herbicides, inorganic herbicides, nitrile herbicides, organophosphorus herbicides, oxadiazolone herbicides, oxazole herbicides, phenoxy herbicides, phenylenediamine herbicides, pyrazole herbicides, pyridazine herbicides, pyridazinone herbicides, pyridine herbicides, pyrimidinediamine herbicides, pyrimidinyloxybenzylamine herbicides, quaternary ammonium herbicides, thiocarbamate herbicides, thiocarbonate herbicides, thiourea herbicides, triazine herbicides, triazinone herbicides, triazole herbicides, triazolone herbicides, triazolopyrimidine herbicides, uracil herbicides, and urea herbicides.. In particular, the rates of use of the added herbicides can be reduced in compositions comprising the polynucleotides. Use rate reductions of the additional added herbicides can be 10-25 percent, 26-50 percent, 51-75 percent or more can be achieved that enhance the activity of the polynucleotides and herbicide composition and is contemplated. Representative herbicides of the families include but are not limited to acetochlor, acifluorfen, acifluorfen-sodium, aclonifen, acrolein, alachlor, alloxydim, allyl alcohol, ametryn, amicarbazone, amidosulfuron, aminopyralid, amitrole, ammonium sulfamate, anilofos, asulam, atraton, atrazine, azimsulfuron, BCPC, beflubutamid, benazolin, benfluralin, benfuresate, bensulfuron, bensulfuron-methyl, bensulide, bentazone, benzfendizone, benzobicyclon, benzofenap, bifenox, bilanafos, bispyribac, bispyribac-sodium, borax, bromacil, bromobutide, bromoxynil, butachlor, butafenacil, butamifos, butralin, butroxydim, butylate, cacodylic acid, calcium chlorate, cafenstrole, carbetamide, carfentrazone, carfentrazone-ethyl, CDEA, CEPC, chlorflurenol, chlorflurenol-methyl, chloridazon, chlorimuron, chlorimuron-ethyl, chloroacetic acid, chlorotoluron, chlorpropham, chlorsulfuron, chlorthal, chlorthal-dimethyl, cinidon-ethyl, cinmethylin, cinosulfuron, cisanilide, clethodim, clodinafop, clodinafop-propargyl, clomazone, clomeprop, clopyralid, cloransulam, cloransulam-methyl, CMA, 4-CPB, CPMF, 4-CPP, CPPC, cresol, cumyluron, cyanamide, cyanazine, cycloate, cyclosulfamuron, cycloxydim, cyhalofop, cyhalofop-butyl, 2,4-D, 3,4-DA, daimuron, dalapon, dazomet, 2,4-DB, 3,4-DB, 2,4-DEB, desmedipham, dicamba, dichlobenil, ortho-dichlorobenzene, para-dichlorobenzene, dichlorprop, dichlorprop-P, diclofop, diclofop-methyl, diclosulam, difenzoquat, difenzoquat metilsulfate, diflufenican, diflufenzopyr, dimefuron, dimepiperate, dimethachlor, dimethametryn, dimethenamid, dimethenamid-P, dimethipin, dimethylarsinic acid, dinitramine, dinoterb, diphenamid, diquat, diquat dibromide, dithiopyr, diuron, DNOC, 3,4-DP, DSMA, EBEP, endothal, EPTC, esprocarb, ethalfluralin, ethametsulfuron, ethametsulfuron-methyl, ethofumesate, ethoxyfen, ethoxysulfuron, etobenzanid, fenoxaprop-P, fenoxaprop-P-ethyl, fentrazamide, ferrous sulfate, flamprop-M, flazasulfuron, florasulam, fluazifop, fluazifop-butyl, fluazifop-P, fluazifop-P-butyl, flucarbazone, flucarbazone-sodium, flucetosulfuron, fluchloralin, flufenacet, flufenpyr, flufenpyr-ethyl, flumetsulam, flumiclorac, flumiclorac-pentyl, flumioxazin, fluometuron, fluoroglycofen, fluoroglycofen-ethyl, flupropanate, flupyrsulfuron, flupyrsulfuron-methyl-sodium, flurenol, fluridone, fluorochloridone, fluoroxypyr, flurtamone, fluthiacet, fluthiacet-methyl, fomesafen, foramsulfuron, fosamine, glufosinate, glufosinate-ammonium, glyphosate, halosulfuron, halosulfuron-methyl, haloxyfop, haloxyfop-P, HC-252, hexazinone, imazamethabenz, imazamethabenz-methyl, imazamox, imazapic, imazapyr, imazaquin, imazethapyr, imazosulfuron, indanofan, iodomethane, iodosulfuron, iodosulfuron-methyl-sodium, ioxynil, isoproturon, isouron, isoxaben, isoxachlortole, isoxaflutole, karbutilate, lactofen, lenacil, linuron, MAA, MAMA, MCPA, MCPA-thioethyl, MCPB, mecoprop, mecoprop-P, mefenacet, mefluidide, mesosulfuron, mesosulfuron-methyl, mesotrione, metam, metamifop, metamitron, metazachlor, methabenzthiazuron, methylarsonic acid, methyldymron, methyl isothiocyanate, metobenzuron, metolachlor, S-metolachlor, metosulam, metoxuron, metribuzin, metsulfuron, metsulfuron-methyl, MK-66, molinate, monolinuron, MSMA, naproanilide, napropamide, naptalam, neburon, nicosulfuron, nonanoic acid, norflurazon, oleic acid (fatty acids), orbencarb, orthosulfamuron, oryzalin, oxadiargyl, oxadiazon, oxasulfuron, oxaziclomefone, oxyfluorfen, paraquat, paraquat dichloride, pebulate, pendimethalin, penoxsulam, pentachlorophenol, pentanochlor, pentoxazone, pethoxamid, petrolium oils, phenmedipham, phenmedipham-ethyl, picloram, picolinafen, pinoxaden, piperophos, potassium arsenite, potassium azide, pretilachlor, primisulfuron, primisulfuron-methyl, prodiamine, profluazol, profoxydim, prometon, prometryn, propachlor, propanil, propaquizafop, propazine, propham, propisochlor, propoxycarbazone, propoxycarbazone-sodium, propyzamide, prosulfocarb, prosulfuron, pyraclonil, pyraflufen, pyraflufen-ethyl, pyrazolynate, pyrazosulfuron, pyrazosulfuron-ethyl, pyrazoxyfen, pyribenzoxim, pyributicarb, pyridafol, pyridate, pyriftalid, pyriminobac, pyriminobac-methyl, pyrimisulfan, pyrithiobac, pyrithiobac-sodium, quinclorac, quinmerac, quinoclamine, quizalofop, quizalofop-P, rimsulfuron, sethoxydim, siduron, simazine, simetryn, SMA, sodium arsenite, sodium azide, sodium chlorate, sulcotrione, sulfentrazone, sulfometuron, sulfometuron-methyl, sulfosate, sulfosulfuron, sulfuric acid, tar oils, 2,3,6-TBA, TCA, TCA-sodium, tebuthiuron, tepraloxydim, terbacil, terbumeton, terbuthylazine, terbutryn, thenylchlor, thiazopyr, thifensulfuron, thifensulfuron-methyl, thiobencarb, tiocarbazil, topramezone, tralkoxydim, tri-allate, triasulfuron, triaziflam, tribenuron, tribenuron-methyl, tricamba, triclopyr, trietazine, trifloxysulfuron, trifloxysulfuron-sodium, trifluralin, triflusulfuron, triflusulfuron-methyl, trihydroxytriazine, tritosulfuron, [3-[2-chloro-4-fluoro-5-(-methyl-6-trifluoromethyl-2,4-dioxo-,2,3,4-t- etrahydropyrimidin-3-yl)phenoxy]-2-pyridyloxy]acetic acid ethyl ester (CAS RN 353292-3-6), 4-[(4,5-dihydro-3-methoxy-4-methyl-5-oxo)-H-,2,4-triazol--ylcarbonyl-sulfamoyl]-5-methylthiophene-3-carboxylic acid (BAY636), BAY747 (CAS RN 33504-84-2), topramezone (CAS RN 2063-68-8), 4-hydroxy-3-[[2-[(2-methoxyethoxy)methyl]-6-(trifluoro-methyl)-3-pyridi- nyl]carbonyl]-bicyclo[3.2.]oct-3-en-2-one (CAS RN 35200-68-5), and 4-hydroxy-3-[[2-(3-methoxypropyl)-6-(difluoromethyl)-3-pyridinyl]carbon-yl]-bicyclo[3.2.]oct-3-en-2-one. Additionally, including herbicidal compounds of unspecified modes of action as described in CN101279950A, CN101279951A, DE10000600A1, DE10116399A1, DE102004054666A1, DE102005014638A1, DE102005014906A1, DE102007012168A1, DE102010042866A1, DE10204951A1, DE10234875A1, DE10234876A1, DE10256353A1, DE10256354A1, DE10256367A1, EP1157991A2, EP1238586A1, EP2147919A1, EP2160098A2, JP03968012B2, JP2001253874A, JP2002080454A, JP2002138075A, JP2002145707A, JP2002220389A, JP2003064059A, JP2003096059A, JP2004051628A, JP2004107228A, JP2005008583A, JP2005239675A, JP2005314407A, JP2006232824A, JP2006282552A, JP2007153847A, JP2007161701A, JP2007182404A, JP2008074840A, JP2008074841A, JP2008133207A, JP2008133218A, JP2008169121A, JP2009067739A, JP2009114128A, JP2009126792A, JP2009137851A, US20060111241A1, US20090036311A1, US20090054240A1, US20090215628A1, US20100099561A1, US20100152443A1, US20110105329A1, US20110201501A1, WO2001055066A2, WO2001056975A1, WO2001056979A1, WO2001090071A2, WO2001090080A1, WO2002002540A1 , WO2002028182A1, WO2002040473A1, WO2002044173A2, WO2003000679A2, WO2003006422A1, WO2003013247A1, WO2003016308A1, WO2003020704A1, WO2003022051A1, WO2003022831A1, WO2003022843A1, WO2003029243A2, WO2003037085A1, WO2003037878A1, WO2003045878A2, WO2003050087A2, WO2003051823A1, WO2003051824A1, WO2003051846A2, WO2003076409A1, WO2003087067A1, WO2003090539A1, WO2003091217A1, WO2003093269A2, WO2003104206A2, WO2004002947A1, WO2004002981A2, WO2004011429A1, WO2004029060A1, WO2004035545A2, WO2004035563A1, WO2004035564A1, WO2004037787A1, WO2004067518A1, WO2004067527A1, WO2004077950A1, WO2005000824A1, WO2005007627A1, WO2005040152A1, WO2005047233A1, WO2005047281A1, WO2005061443A2, WO2005061464A1, WO2005068434A1, WO2005070889A1, WO2005089551A1, WO2005095335A1, WO2006006569A1, WO2006024820A1, WO2006029828A1, WO2006029829A1, WO2006037945A1, WO2006050803A1, WO2006090792A1, WO2006123088A2, WO2006125687A1, WO2006125688A1, WO2007003294A1, WO2007026834A1, WO2007071900A1, WO2007077201A1, WO2007077247A1, WO2007096576A1, WO2007119434A1, WO2007134984A1, WO2008009908A1, WO2008029084A1, WO2008059948A1, WO2008071918A1, WO2008074991A1, WO2008084073A1, WO2008100426A2, WO2008102908A1, WO2008152072A2, WO2008152073A2, WO2009000757A1, WO2009005297A2, WO2009035150A2, WO2009063180A1, WO2009068170A2, WO2009068171A2, WO2009086041A1, WO2009090401A2, WO2009090402A2, WO2009115788A1, WO2009116558A1, WO2009152995A1, WO2009158258A1, WO2010012649A1, WO2010012649A1, WO2010026989A1, WO2010034153A1, WO2010049270A1, WO2010049369A1, WO2010049405A1, WO2010049414A1, WO2010063422A1, WO2010069802A1, WO2010078906A2, WO2010078912A1, WO2010104217A1, WO2010108611A1, WO2010112826A3, WO2010116122A3, WO2010119906A1, WO2010130970A1, WO2011003776A2, WO2011035874A1, WO2011065451A1

The trigger polynucleotide and oligonucleotide molecule compositions are useful in compositions, such as liquids that comprise the polynucleotide molecules at low concentrations, alone or in combination with other components, for example one or more herbicide molecules, either in the same solution or in separately applied liquids that also provide a transfer agent. While there is no upper limit on the concentrations and dosages of polynucleotide molecules that can useful in the methods, lower effective concentrations and dosages will generally be sought for efficiency. The concentrations can be adjusted in consideration of the volume of spray or treatment applied to plant leaves or other plant part surfaces, such as flower petals, stems, tubers, fruit, anthers, pollen, or seed. In one embodiment, a useful treatment for herbaceous plants using 25-mer oligonucleotide molecules is about 1 nanomole (nmol) of oligonucleotide molecules per plant, for example, from about 0.05 to 1 nmol per plant. Other embodiments for herbaceous plants include useful ranges of about 0.05 to about 100 nmol, or about 0.1 to about 20 nmol, or about 1 nmol to about 10 nmol of polynucleotides per plant. Very large plants, trees, or vines may require correspondingly larger amounts of polynucleotides. When using long dsRNA molecules that can be processed into multiple oligonucleotides, lower concentrations can be used. To illustrate embodiments, the factor 1X, when applied to oligonucleotide molecules is arbitrarily used to denote a treatment of 0.8 nmol of polynucleotide molecule per plant; 10X, 8 nmol of polynucleotide molecule per plant; and 100X, 80 nmol of polynucleotide molecule per plant.

The polynucleotide compositions are useful in compositions, such as liquids that comprise polynucleotide molecules, alone or in combination with other components either in the same liquid or in separately applied liquids that provide a transfer agent. As used herein, a transfer agent is an agent that, when combined with a polynucleotide in a composition that is topically applied to a target plant surface, enables the polynucleotide to enter a plant cell. In certain embodiments, a transfer agent is an agent that conditions the surface of plant tissue, e. g., leaves, stems, roots, flowers, or fruits, to permeation by the polynucleotide molecules into plant cells. The transfer of polynucleotides into plant cells can be facilitated by the prior or contemporaneous application of a polynucleotide-transferring agent to the plant tissue. In some embodiments the transferring agent is applied subsequent to the application of the polynucleotide composition. The polynucleotide transfer agent enables a pathway for polynucleotides through cuticle wax barriers, stomata and/or cell wall or membrane barriers into plant cells. Suitable transfer agents to facilitate transfer of the polynucleotide into a plant cell include agents that increase permeability of the exterior of the plant or that increase permeability of plant cells to oligonucleotides or polynucleotides. Such agents to facilitate transfer of the composition into a plant cell include a chemical agent, or a physical agent, or combinations thereof. Chemical agents for conditioning or transfer include (a) surfactants, (b) an organic solvent or an aqueous solution or aqueous mixtures of organic solvents, (c) oxidizing agents, (d) acids, (e) bases, (f) oils, (g) enzymes, or combinations thereof. Embodiments of the method can optionally include an incubation step, a neutralization step (e.g., to neutralize an acid, base, or oxidizing agent, or to inactivate an enzyme), a rinsing step, or combinations thereof. Embodiments of agents or treatments for conditioning of a plant to permeation by polynucleotides include emulsions, reverse emulsions, liposomes, and other micellar-like compositions. Embodiments of agents or treatments for conditioning of a plant to permeation by polynucleotides include counter-ions or other molecules that are known to associate with nucleic acid molecules, *e.g.,* inorganic ammonium ions, alkyl ammonium ions, lithium ions, polyamines such as spermine, spermidine, or putrescine, and other cations. Organic solvents useful in conditioning a plant to permeation by polynucleotides include DMSO, DMF, pyridine, *N*-pyrrolidine, hexamethylphosphoramide, acetonitrile, dioxane, polypropylene glycol, other solvents miscible with water or that will dissolve phosphonucleotides in non-aqueous systems (such as is used in synthetic reactions). Naturally derived or synthetic oils with or without surfactants or emulsifiers can be used, *e. g*., plant-sourced oils, crop oils (such as those listed in the 9^{th} Compendium of Herbicide Adjuvants, publicly available on the worldwide web (internet) at herbicide.adjuvants.com can be used, *e.g.,* paraffinic oils, polyol fatty acid esters, or oils with short-chain molecules modified with amides or polyamines such as polyethyleneimine or *N*-pyrrolidine. Transfer agents include, but are not limited to, organosilicone preparations.

An agronomic field in need of plant control is treated by application of the composition of the present invention directly to the surface of the growing plants, such as by a spray. For example, the method is applied to control weeds in a field of crop plants by spraying the field with the composition.. The composition can be provided as a tank mix, a sequential treatment of components (generally the polynucleotide containing composition followed by the herbicide), or a simultaneous treatment or mixing of one or more of the components of the composition from separate containers. Treatment of the field can occur as often as needed to provide weed control and the components of the composition can be adjusted to target specific weed species or weed families through utilization of specific polynucleotides or polynucleotide compositions capable of selectively targeting the specific species or plant family to be controlled. The composition can be applied at effective use rates according to the time of application to the field, for example, preplant, at planting, post planting, post harvest. ALS inhibitor herbicides can be applied to a field at rates of 3 to 150 g ai/ha or more. The polynucleotides of the composition can be applied at rates of 1 to 30 grams per acre depending on the number of trigger molecules needed for the scope of weeds in the field.

Crop plants in which weed control is needed include but are not limited to, corn, soybean, cotton, canola, sugar beet, alfalfa, sugarcane, rice, and wheat; ii) vegetable plants including, but not limited to, tomato, sweet pepper, hot pepper, melon, watermelon, cucumber, eggplant, cauliflower, broccoli, lettuce, spinach, onion, peas, carrots, sweet corn, Chinese cabbage, leek, fennel, pumpkin, squash or gourd, radish, Brussels sprouts, tomatillo, garden beans, dry beans, or okra; iii) culinary plants including, but not limited to, basil, parsley, coffee, or tea; or, iv) fruit plants including but not limited to apple, pear, cherry, peach, plum, apricot, banana, plantain, table grape, wine grape, citrus, avocado, mango, or berry; v) a tree grown for ornamental or commercial use, including, but not limited to, a fruit or nut tree; or, vi) an ornamental plant (e. g., an ornamental flowering plant or shrub or turf grass). The methods and compositions provided herein can also be applied to plants produced by a cutting, cloning, or grafting process (i. e., a plant not grown from a seed) include fruit trees and plants that include, but are not limited to, citrus, apples, avocados, tomatoes, eggplant, cucumber, melons, watermelons, and grapes as well as various ornamental plants.

### Pesticidal Mixtures

The polynucleotide compositions may also be used as mixtures with various agricultural chemicals and/or insecticides, miticides and fungicides, pesticidal and biopesticidal agents. Examples include but are not limited to azinphos-methyl, acephate, isoxathion, isofenphos, ethion, etrimfos, oxydemeton-methyl, oxydeprofos, quinalphos, chlorpyrifos, chlorpyrifos-methyl, chlorfenvinphos, cyanophos, dioxabenzofos, dichlorvos, disulfoton, dimethylvinphos, dimethoate, sulprofos, diazinon, thiometon, tetrachlorvinphos, temephos, tebupirimfos, terbufos, naled, vamidothion, pyraclofos, pyridafenthion, pirimiphos-methyl, fenitrothion, fenthion, phenthoate, flupyrazophos, prothiofos, propaphos, profenofos, phoxime, phosalone, phosmet, formothion, phorate, malathion, mecarbam, mesulfenfos, methamidophos, methidathion, parathion, methyl parathion, monocrotophos, trichlorphon, EPN, isazophos, isamidofos, cadusafos, diamidaphos, dichlofenthion, thionazin, fenamiphos, fosthiazate, fosthietan, phosphocarb, DSP, ethoprophos, alanycarb, aldicarb, isoprocarb, ethiofencarb, carbaryl, carbosulfan, xylylcarb, thiodicarb, pirimicarb, fenobucarb, furathiocarb, propoxur, bendiocarb, benfuracarb, methomyl, metolcarb, XMC, carbofuran, aldoxycarb, oxamyl, acrinathrin, allethrin, esfenvalerate, empenthrin, cycloprothrin, cyhalothrin, gamma-cyhalothrin, lambda-cyhalothrin, cyfluthrin, beta-cyfluthrin, cypermethrin, alpha-cypermethrin, zeta-cypermethrin, silafluofen, tetramethrin, tefluthrin, deltamethrin, tralomethrin, bifenthrin, phenothrin, fenvalerate, fenpropathrin, furamethrin, prallethrin, flucythrinate, fluvalinate, flubrocythrinate, permethrin, resmethrin, ethofenprox, cartap, thiocyclam, bensultap, acetamiprid, imidacloprid, clothianidin, dinotefuran, thiacloprid, thiamethoxam, nitenpyram, chlorfluazuron, diflubenzuron, teflubenzuron, triflumuron, novaluron, noviflumuron, bistrifluoron, fluazuron, flucycloxuron, flufenoxuron, hexaflumuron, lufenuron, chromafenozide, tebufenozide, halofenozide, methoxyfenozide, diofenolan, cyromazine, pyriproxyfen, buprofezin, methoprene, hydroprene, kinoprene, triazamate, endosulfan, chlorfenson, chlorobenzilate, dicofol, bromopropylate, acetoprole, fipronil, ethiprole, pyrethrin, rotenone, nicotine sulphate, BT (Bacillus Thuringiensis) agent, spinosad, abamectin, acequinocyl, amidoflumet, amitraz, etoxazole, chinomethionat, clofentezine, fenbutatin oxide, dienochlor, cyhexatin, spirodiclofen, spiromesifen, tetradifon, tebufenpyrad, binapacryl, bifenazate, pyridaben, pyrimidifen, fenazaquin, fenothiocarb, fenpyroximate, fluacrypyrim, fluazinam, flufenzin, hexythiazox, propargite, benzomate, polynactin complex, milbemectin, lufenuron, mecarbam, methiocarb, mevinphos, halfenprox, azadirachtin, diafenthiuron, indoxacarb, emamectin benzoate, potassium oleate, sodium oleate, chlorfenapyr, tolfenpyrad, pymetrozine, fenoxycarb, hydramethylnon, hydroxy propyl starch, pyridalyl, flufenerim, flubendiamide, flonicamid, metaflumizole, lepimectin, TPIC, albendazole, oxibendazole, oxfendazole, trichlamide, fensulfothion, fenbendazole, levamisole hydrochloride, morantel tartrate, dazomet, metam-sodium, triadimefon, hexaconazole, propiconazole, ipconazole, prochloraz, triflumizole, tebuconazole, epoxiconazole, difenoconazole, flusilazole, triadimenol, cyproconazole, metconazole, fluquinconazole, bitertanol, tetraconazole, triticonazole, flutriafol, penconazole, diniconazole, fenbuconazole, bromuconazole, imibenconazole, simeconazole, myclobutanil, hymexazole, imazalil, furametpyr, thifluzamide, etridiazole, oxpoconazole, oxpoconazole fumarate, pefurazoate, prothioconazole, pyrifenox, fenarimol, nuarimol, bupirimate, mepanipyrim, cyprodinil, pyrimethanil, metalaxyl, mefenoxam, oxadixyl, benalaxyl, thiophanate, thiophanate-methyl, benomyl, carbendazim, fuberidazole, thiabendazole, manzeb, propineb, zineb, metiram, maneb, ziram, thiuram, chlorothalonil, ethaboxam, oxycarboxin, carboxin, flutolanil, silthiofam, mepronil, dimethomorph, fenpropidin, fenpropimorph, spiroxamine, tridemorph, dodemorph, flumorph, azoxystrobin, kresoxim-methyl, metominostrobin, orysastrobin, fluoxastrobin, trifloxystrobin, dimoxystrobin, pyraclostrobin, picoxystrobin, iprodione, procymidone, vinclozolin, chlozolinate, flusulfamide, dazomet, methyl isothiocyanate, chloropicrin, methasulfocarb, hydroxyisoxazole, potassium hydroxyisoxazole, echlomezol, D-D, carbam, basic copper chloride, basic copper sulfate, copper nonylphenolsulfonate, oxine copper, DBEDC, anhydrous copper sulfate, copper sulfate pentahydrate, cupric hydroxide, inorganic sulfur, wettable sulfur, lime sulfur, zinc sulfate, fentin, sodium hydrogen carbonate, potassium hydrogen carbonate, sodium hypochlorite, silver, edifenphos, tolclofos-methyl, fosetyl, iprobenfos, dinocap, pyrazophos, carpropamid, fthalide, tricyclazole, pyroquilon, diclocymet, fenoxanil, kasugamycin, validamycin, polyoxins, blasticiden S, oxytetracycline, mildiomycin, streptomycin, rape seed oil, machine oil, benthiavalicarbisopropyl, iprovalicarb, propamocarb, diethofencarb, fluoroimide, fludioxanil, fenpiclonil, quinoxyfen, oxolinic acid, chlorothalonil, captan, folpet, probenazole, acibenzolar-S-methyl, tiadinil, cyflufenamid, fenhexamid, diflumetorim, metrafenone, picobenzamide, proquinazid, famoxadone, cyazofamid, fenamidone, zoxamide, boscalid, cymoxanil, dithianon, fluazinam, dichlofluanide, triforine, isoprothiolane, ferimzone, diclomezine, tecloftalam, pencycuron, chinomethionat, iminoctadine acetate, iminoctadine albesilate, ambam, polycarbamate, thiadiazine, chloroneb, nickel dimethyldithiocarbamate, guazatine, dodecylguanidine-acetate, quintozene, tolylfluanid, anilazine, nitrothalisopropyl, fenitropan, dimethirimol, benthiazole, harpin protein, flumetover, mandipropamide and penthiopyrad.

### Polynucleotides

As used herein, the term "DNA", "DNA molecule", "DNA polynucleotide molecule" refers to a single-stranded DNA (ssDNA) or double-stranded DNA (dsDNA) molecule of genomic or synthetic origin, such as, a polymer of deoxyribonucleotide bases or a DNA polynucleotide molecule. As used herein, the term "DNA sequence", "DNA nucleotide sequence" or "DNA polynucleotide sequence" refers to the nucleotide sequence of a DNA molecule. As used herein, the term "RNA", "RNA molecule", "RNA polynucleotide molecule" refers to a single-stranded RNA (ssRNA) or double-stranded RNA (dsRNA) molecule of genomic or synthetic origin, such as, a polymer of ribonucleotide bases that comprise single or double stranded regions. Unless otherwise stated, nucleotide sequences in the text of this specification are given, when read from left to right, in the 5' to 3' direction. The nomenclature used herein is that required by Title 37 of the United States Code of Federal Regulations § 1.822 and set forth in the tables in WIPO Standard ST.25 (1998), Appendix 2, Tables 1 and 3.

As used herein, "polynucleotide" refers to a DNA or RNA molecule containing multiple nucleotides and generally refers both to "oligonucleotides" (a polynucleotide molecule of typically 50 or fewer nucleotides in length) and polynucleotides of 51 or more nucleotides. Embodiments of this invention include compositions including oligonucleotides having a length of 18-25 nucleotides (18-mers, 19-mers, 20-mers, 21-mers, 22-mers, 23-mers, 24-mers, or 25-mers), for example, oligonucleotides SEQ ID NO: 1364-1691 and 4167-4201 or fragments thereof, or medium-length polynucleotides having a length of 26 or more nucleotides (polynucleotides of 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, about 65, about 70, about 75, about 80, about 85, about 90, about 95, about 100, about 110, about 120, about 130, about 140, about 150, about 160, about 170, about 180, about 190, about 200, about 210, about 220, about 230, about 240, about 250, about 260, about 270, about 280, about 290, or about 300 nucleotides), for example, oligonucleotides SEQ ID NO: 46-1363 and 1789-4166 or fragments thereof or long polynucleotides having a length greater than about 300 nucleotides (for example, polynucleotides of between about 300 to about 400 nucleotides, between about 400 to about 500 nucleotides, between about 500 to about 600 nucleotides, between about 600 to about 700 nucleotides, between about 700 to about 800 nucleotides, between about 800 to about 900 nucleotides, between about 900 to about 1000 nucleotides, between about 300 to about 500 nucleotides, between about 300 to about 600 nucleotides, between about 300 to about 700 nucleotides, between about 300 to about 800 nucleotides, between about 300 to about 900 nucleotides, or about 1000 nucleotides in length, or even greater than about 1000 nucleotides in length, for example up to the entire length of a target gene including coding or non-coding or both coding and non-coding portions of the target gene) for example, polynucleotides of Table 1 (SEQ ID NO:1-45 and 1692-1788), wherein the selected polynucleotides or fragments thereof are homologous or complementary to SEQ ID NO:1-45 and 1692-1788 and suppresses, represses or otherwise delay the expression of the target ALS gene.. A target gene comprises any polynucleotide molecule in a plant cell or fragment thereof for which the modulation of the expression of the target gene is provided by the methods and compositions of the present invention. Where a polynucleotide is double-stranded, its length can be similarly described in terms of base pairs. Oligonucleotides and polynucleotides of the present invention can be made that are essentially identical or essentially complementary to adjacent genetic elements of a gene, for example, spanning the junction region of an intron and exon, the junction region of a promoter and a transcribed region, the junction region of a 5' leader and a coding sequence, the junction of a 3' untranslated region and a coding sequence.

Polynucleotide compositions used in the various embodiments of this invention include compositions including oligonucleotides or polynucleotides or a mixture of both, including RNA or DNA or RNA/DNA hybrids or chemically modified oligonucleotides or polynucleotides or a mixture thereof. In some embodiments, the polynucleotide may be a combination of ribonucleotides and deoxyribonucleotides, for example, synthetic polynucleotides consisting mainly of ribonucleotides but with one or more terminal deoxyribonucleotides or synthetic polynucleotides consisting mainly of deoxyribonucleotides but with one or more terminal dideoxyribonucleotides. In some embodiments, the polynucleotide includes non-canonical nucleotides such as inosine, thiouridine, or pseudouridine. In some embodiments, the polynucleotide includes chemically modified nucleotides. Examples of chemically modified oligonucleotides or polynucleotides are well known in the art; see, for example, US Patent Publication 20110171287, US Patent Publication 20110171176, and US Patent Publication 20110152353, US Patent Publication, 20110152346, US Patent Publication 20110160082. For example, including but not limited to the naturally occurring phosphodiester backbone of an oligonucleotide or polynucleotide can be partially or completely modified with phosphorothioate, phosphorodithioate, or methylphosphonate internucleotide linkage modifications, modified nucleoside bases or modified sugars can be used in oligonucleotide or polynucleotide synthesis, and oligonucleotides or polynucleotides can be labeled with a fluorescent moiety (for example, fluorescein or rhodamine) or other label (for example, biotin).

The polynucleotides can be single- or double-stranded RNA or single- or double-stranded DNA or double-stranded DNA/RNA hybrids or modified analogues thereof, and can be of oligonucleotide lengths or longer. In more specific embodiments of the invention the polynucleotides that provide single-stranded RNA in the plant cell are selected from the group consisting of (a) a single-stranded RNA molecule (ssRNA), (b) a single-stranded RNA molecule that self-hybridizes to form a double-stranded RNA molecule, (c) a double-stranded RNA molecule (dsRNA), (d) a single-stranded DNA molecule (ssDNA), (e) a single-stranded DNA molecule that self-hybridizes to form a double-stranded DNA molecule, and (f) a single-stranded DNA molecule including a modified Pol III gene that is transcribed to an RNA molecule, (g) a double-stranded DNA molecule (dsDNA), (h) a double-stranded DNA molecule including a modified Pol III gene that is transcribed to an RNA molecule, (i) a double-stranded, hybridized RNA/DNA molecule, or combinations thereof. In some embodiments these polynucleotides include chemically modified nucleotides or non-canonical nucleotides. In embodiments of the method the polynucleotides include double-stranded DNA formed by intramolecular hybridization, double-stranded DNA formed by intermolecular hybridization, double-stranded RNA formed by intramolecular hybridization, or double-stranded RNA formed by intermolecular hybridization. In one embodiment the polynucleotides include single-stranded DNA or single-stranded RNA that self-hybridizes to form a hairpin structure having an at least partially double-stranded structure including at least one segment that will hybridize to RNA transcribed from the gene targeted for suppression. Not intending to be bound by any mechanism, it is believed that such polynucleotides are or will produce single-stranded RNA with at least one segment that will hybridize to RNA transcribed from the gene targeted for suppression. In certain other embodiments the polynucleotides further includes a promoter, generally a promoter functional in a plant, for example, a pol II promoter, a pol III promoter, a pol IV promoter, or a pol V promoter.

The term "gene" refers to components that comprise chromosomal DNA, plasmid DNA, cDNA, intron and exon DNA, artificial DNA polynucleotide, or other DNA that encodes a peptide, polypeptide, protein, or RNA transcript molecule, and the genetic elements flanking the coding sequence that are involved in the regulation of expression, such as, promoter regions, 5' leader regions, 3' untranslated region that may exist as native genes or transgenes in a plant genome. The gene or a fragment thereof is isolated and subjected to polynucleotide sequencing methods that determines the order of the nucleotides that comprise the gene. Any of the components of the gene are potential targets for a trigger oligonucleotide and polynucleotides..

The trigger polynucleotide molecules are designed to modulate expression by inducing regulation or suppression of an endogenous ALS gene in a plant and are designed to have a nucleotide sequence essentially identical or essentially complementary to the nucleotide sequence of an endogenous ALS gene of a plant or to the sequence of RNA transcribed from an endogenous ALS gene of a plant, including a transgene in a plant that provides for a herbicide resistant ALS enzyme, which can be coding sequence or non-coding sequence. Effective molecules that modulate expression are referred to as "a trigger molecule, or trigger polynucleotide". By "essentially identical" or "essentially complementary" is meant that the trigger polynucleotides (or at least one strand of a double-stranded polynucleotide or portion thereof, or a portion of a single strand polynucleotide) are designed to hybridize to the endogenous gene noncoding sequence or to RNA transcribed (known as messenger RNA or an RNA transcript) from the endogenous gene to effect regulation or suppression of expression of the endogenous gene. Trigger molecules are identified by "tiling" the gene targets with partially overlapping probes or non-overlapping probes of antisense or sense polynucleotides that are essentially identical or essentially complementary to the nucleotide sequence of an endogenous gene. Multiple target sequences can be aligned and sequence regions with homology in common, according to the methods of the present invention, are identified as potential trigger molecules for the multiple targets. Multiple trigger molecules of various lengths, for example 18-25 nucleotides, 26-50 nucleotides, 51-100 nucleotides, 101-200 nucleotides, 201-300 nucleotides or more can be pooled into a few treatments in order to investigate polynucleotide molecules that cover a portion of a gene sequence (for example, a portion of a coding versus a portion of a noncoding region, or a 5' versus a 3' portion of a gene) or an entire gene sequence including coding and noncoding regions of a target gene. Polynucleotide molecules of the pooled trigger molecules can be divided into smaller pools or single molecules inorder to identify trigger molecules that provide the desired effect.

The target gene RNA and DNA polynucleotide molecules (Table 1, SEQ ID NO:1-45 and 1692-1788) are sequenced by any number of available methods and equipment. Some of the sequencing technologies are available commercially, such as the sequencing-by-hybridization platform from Affymetrix Inc. (Sunnyvale, Calif.) and the sequencing-by-synthesis platforms from 454 Life Sciences (Bradford, Conn.), Illumina/Solexa (Hayward, Calif.) and Helicos Biosciences (Cambridge, Mass.), and the sequencing-by-ligation platform from Applied Biosystems (Foster City, Calif.), as described below. In addition to the single molecule sequencing performed using sequencing-by-synthesis of Helicos Biosciences, other single molecule sequencing technologies are encompassed and include the SMRT™. technology of Pacific Biosciences, the Ion Torrent™, technology, and nanopore sequencing being developed for example, by Oxford Nanopore Technologies. An ALStarget gene comprising DNA or RNA can be isolated using primers or probes essentially complementary or essentially homologous to SEQ ID NO:1-45 and 1692-1788 or a fragment thereof. A polymerase chain reaction (PCR) gene fragment can be produced using primers essentially complementary or essentially homologous to SEQ ID NO:1-45 and 1692-1788 or a fragment thereof that is useful to isolate an ALS gene from a plant genome. SEQ ID NO: 1-45 and 1692-1788 or fragments thereof can be used in various sequence capture technologies to isolate additional target gene sequences, for example, including but not limited to Roche NimbleGen® (Madison, WI) and Streptavdin-coupled Dynabeads® (Life Technologies, Grand Island, NY) and US20110015084.

Embodiments of functional single-stranded polynucleotides have sequence complementarity that need not be 100 percent, but is at least sufficient to permit hybridization to RNA transcribed from the target gene or DNA of the target gene to form a duplex to permit a gene silencing mechanism. Thus, in embodiments, a polynucleotide fragment is designed to be essentially identical to, or essentially complementary to, a sequence of 18 or more contiguous nucleotides in either the target ALS gene sequence or messenger RNA transcribed from the target gene. By "essentially identical" is meant having 100 percent sequence identity or at least about 83, 84, 85, 86, 87, 88, 89, 90, 91, 92, 93, 94, 95, 96, 97, 98, or 99 percent sequence identity when compared to the sequence of 18 or more contiguous nucleotides in either the target gene or RNA transcribed from the target gene; by "essentially complementary" is meant having 100 percent sequence complementarity or at least about 83, 84, 85, 86, 87, 88, 89, 90, 91, 92, 93, 94, 95, 96, 97, 98, or 99 percent sequence complementarity when compared to the sequence of 18 or more contiguous nucleotides in either the target gene or RNA transcribed from the target gene. In some embodiments, polynucleotide molecules are designed to have 100 percent sequence identity with or complementarity to one allele or one family member of a given target gene (coding or non-coding sequence of a gene for of the present invention); in other embodiments the polynucleotide molecules are designed to have 100 percent sequence identity with or complementarity to multiple alleles or family members of a given target gene.

"Identity" refers to the degree of similarity between two polynucleic acid or protein sequences. An alignment of the two sequences is performed by a suitable computer program. A widely used and accepted computer program for performing sequence alignments is CLUSTALW v1.6 (Thompson, et al. Nucl. Acids Res., 22: 4673-4680, 1994). The number of matching bases or amino acids is divided by the total number of bases or amino acids, and multiplied by 100 to obtain a percent identity. For example, if two 580 base pair sequences had 145 matched bases, they would be 25 percent identical. If the two compared sequences are of different lengths, the number of matches is divided by the shorter of the two lengths. For example, if there are 100 matched amino acids between a 200 and a 400 amino acid protein, they are 50 percent identical with respect to the shorter sequence. If the shorter sequence is less than 150 bases or 50 amino acids in length, the number of matches are divided by 150 (for nucleic acid bases) or 50 (for amino acids), and multiplied by 100 to obtain a percent identity.

Trigger molecules for specific gene family members can be identified from coding and/or non-coding sequences of gene families of a plant or multiple plants, by aligning and selecting 200-300 polynucleotide fragments from the least homologous regions amongst the aligned sequences and evaluated using topically applied polynucleotides (as sense or anti-sense ssDNA or ssRNA, dsRNA, or dsDNA) to determine their relative effectiveness in inducing the herbicidal phenotype. The effective segments are further subdivided into 50-60 polynucleotide fragments, prioritized by least homology, and reevaluated using topically applied polynucleotides. The effective 50-60 polynucleotide fragments are subdivided into 19-30 polynucleotide fragments, prioritized by least homology, and again evaluated for induction of the herbicidal phenotype. Once relative effectiveness is determined, the fragments are utilized singly, or again evaluated in combination with one or more other fragments to determine the trigger composition or mixture of trigger polynucleotides for providing the herbicidal phenotype.

Trigger molecules for broad activity can be identified from coding and/or non-coding sequences of gene families of a plant or multiple plants, by aligning and selecting 200-300 polynucleotide fragments from the most homologous regions amongst the aligned sequences and evaluated using topically applied polynucleotides (as sense or anti-sense ssDNA or ssRNA, dsRNA, or dsDNA) to determine their relative effectiveness in inducing the herbicidal phenotype. The effective segments are subdivided into 50-60 polynucleotide fragments, prioritized by most homology, and reevaluated using topically applied polynucleotides. The effective 50-60 polynucleotide fragments are subdivided into 19-30 polynucleotide fragments, prioritized by most homology, and again evaluated for induction of the herbicidal phenotype. Once relative effectiveness is determined, the fragments may be utilized singly, or in combination with one or more other fragments to determine the trigger composition or mixture of trigger polynucleotides for providing the herbicidal phenotype.

Methods of making polynucleotides are well known in the art. Chemical synthesis, in vivo synthesis and in vitro synthesis methods and compositions are known in the art and include various viral elements, microbial cells, modified polymerases, and modified nucleotides. Commercial preparation of oligonucleotides often provides two deoxyribonucleotides on the 3' end of the sense strand. Long polynucleotide molecules can be synthesized from commercially available kits, for example, kits from Applied Biosystems/Ambion (Austin, TX) have DNA ligated on the 5' end in a microbial expression cassette that includes a bacterial T7 polymerase promoter that makes RNA strands that can be assembled into a dsRNA and kits provided by vaious manufacturers that include T7 RiboMax Express (Promega, Madison, WI), AmpliScribe T7-Flash (Epicentre, Madison, WI), and TranscriptAid T7 High Yield (Fermentas, Glen Burnie, MD). dsRNA molecules can be produced from microbial expression cassettes in bacterial cells (Ongvarrasopone et al. ScienceAsia 33:35-39; Yin, Appl. Microbiol. Biotechnol 84:323-333, 2009; Liu et al., BMC Biotechnology 10:85, 2010) that have regulated or deficient RNase III enzyme activity or the use of various viral vectors to produce sufficient quantities of dsRNA. ALS gene fragments are inserted into the microbial expression cassettes in a position in which the fragments are express to produce ssRNA or dsRNA useful in the methods described herein to regulate expression on a target ALS gene. Long polynucleotide molecules can also be assembled from multiple RNA or DNA fragments. In some embodiments design parameters such as Reynolds score (Reynolds et al. Nature Biotechnology 22, 326 - 330 (2004),Tuschl rules (Pei and Tuschl, Nature Methods 3(9): 670-676, 2006), i-score (Nucleic Acids Res 35: e123, 2007), i-Score Designer tool and associated algorithms (Nucleic Acids Res 32: 936-948, 2004. Biochem Biophys Res Commun 316: 1050-1058, 2004, Nucleic Acids Res 32: 893-901, 2004, Cell Cycle 3: 790-5, 2004, Nat Biotechnol 23: 995-1001, 2005, Nucleic Acids Res 35: e27, 2007, BMC Bioinformatics 7: 520, 2006, Nucleic Acids Res 35: e123, 2007, Nat Biotechnol 22: 326-330, 2004) are known in the art and may be used in selecting polynucleotide sequences effective in gene silencing. In some embodiments the sequence of a polynucleotide is screened against the genomic DNA of the intended plant to minimize unintentional silencing of other genes.

Ligands can be tethered to a polynucleotide, for example a dsRNA, ssRNA, dsDNA or ssDNA. Ligands in general can include modifiers, e.g., for enhancing uptake; diagnostic compounds or reporter groups e.g., for monitoring distribution; cross-linking agents; nuclease-resistance conferring moieties; and natural or unusual nucleobases. General examples include lipophiles, lipids (e.g., cholesterol, a bile acid, or a fatty acid (e.g., lithocholic-oleyl, lauroyl, docosnyl, stearoyl, palmitoyl, myristoyl oleoyl, linoleoyl), steroids (e.g., uvaol, hecigenin, diosgenin), terpenes (e.g., triterpenes, e.g., sarsasapogenin, Friedelin, epifriedelanol derivatized lithocholic acid), vitamins (e.g., folic acid, vitamin A, biotin, pyridoxal), carbohydrates, proteins, protein binding agents, integrin targeting molecules, polycationics, peptides, polyamines, and peptide mimics. The ligand may also be a recombinant or synthetic molecule, such as a synthetic polymer, e.g., polyethylene glycol (PEG), PEG-40K, PEG-20K and PEG-5K. Other examples of ligands include lipophilic molecules, e.g, cholesterol, cholic acid, adamantane acetic acid, 1-pyrene butyric acid, dihydrotestosterone, glycerol (e.g., esters and ethers thereof, e.g., C.sub.10, C.sub.11, C.sub.12, C.sub.13, C.sub.14, C.sub.15, C.sub.16, C.sub.17, C.sub.18, C.sub.19, or C.sub.20 alkyl; e.g., lauroyl, docosnyl, stearoyl, oleoyl, linoleoyl 1,3-bis-O(hexadecyl)glycerol, 1,3-bis-O(octaadecyl)glycerol), geranyloxyhexyl group, hexadecylglycerol, borneol, menthol, 1,3-propanediol, heptadecyl group, palmitic acid, myristic acid, O3-(oleoyl)lithocholic acid, O3-(oleoyl)cholenic acid, dodecanoyl, lithocholyl, 5.beta.-cholanyl, N,N-distearyl-lithocholamide, 1,2-di-O-stearoylglyceride, dimethoxytrityl, or phenoxazine) and PEG (e.g., PEG-5K, PEG-20K, PEG-40K). Preferred lipophilic moieties include lipid, cholesterols, oleyl, retinyl, or cholesteryl residues.

Conjugating a ligand to a dsRNA can enhance its cellular absorption, lipophilic compounds that have been conjugated to oligonucleotides include 1-pyrene butyric acid, 1,3-bis-O-(hexadecyl)glycerol, and menthol. One example of a ligand for receptor-mediated endocytosis is folic acid. Folic acid enters the cell by folate-receptor-radiated endocytosis. dsRNA compounds bearing folic acid would be efficiently transported into the cell via the folate-receptor-mediated endocytosis. Other ligands that have been conjugated to oligonucleotides include polyethylene glycols, carbohydrate clusters, cross-linking agents, porphyrin conjugates, delivery peptides and lipids such as cholesterol. In certain instances, conjugation of a cationic ligand to oligonucleotides results in improved resistance to nucleases. Representative examples of cationic ligands are propylammonium and dimethylpropylammonium. Interestingly, antisense oligonucleotides were reported to retain their high binding affinity to mRNA when the cationic ligand was dispersed, throughout the oligonucleotide. See M. Manoharan Antisense & Nucleic Acid Drug Development 2002, 12, 103 and references therein.

A biologic delivery can be accomplished by a variety of methods including, without limitation, (1) loading liposomes with a dsRNA acid molecule provided herein and (2) complexing a dsRNA molecule with lipids or liposomes to form nucleic acid-lipid or nucleic acid-liposome complexes. The liposome can be composed of cationic and neutral lipids commonly used to transfect cells in vitro. Cationic lipids can complex (e.g., charge-associate) with negatively charged, nucleic acids to form liposomes. Examples of cationic liposomes include, without limitation, lipofectin, lipofectamine, lipofectace, and DOTAP. Procedures for forming liposomes are well known in the art. Liposome compositions can be formed, for example, from phosphatidylcholine, dimyristoyl phosphatidylcholine, dipalmitoyl phosphatidylcholine, dimyristoyl phosphatidyl glycerol, dioleoyl phosphatidylethanolamine or liposomes comprising dihydrosphingomyelin (DHSM) Numerous lipophilic agents are commercially available, including Lipofectin® (Invitrogen/Life Technologies, Carlsbad, Calif.) and Effectene™ (Qiagen, Valencia, Calif.), In addition, systemic delivery methods can be optimized using commercially available cationic lipids such as DDAB or DOTAP, each of which can be mixed with a neutral lipid such as DOPE or cholesterol. In some eases, liposomes such as those described by Templeton et al. (Nature Biotechnology, 15:647-652 (1997)) can be used. In other embodiments, polycations such as polyethyleneimine can be used to achieve delivery in vivo and ex vivo (Boletta et al., J. Am Soc. Nephrol. 7:1728 (1996)). Additional information regarding the use of liposomes to deliver nucleic acids can be found in U.S. Pat. No. 6,271,359, PCT Publication WO 96/40964 and Morrissey, D. et al. 2005. Nat Biotechnol. 23(8):1002-7.

In certain embodiments, an organosilicone preparation that is commercially available as Silwet® L-77 surfactant having CAS Number 27306-78-1 and EPA Number: CAL.REG.NO. 5905-50073-AA, and currently available from Momentive Performance Materials, Albany, New York can be used to prepare a polynucleotide composition. In certain embodiments where a Silwet L-77 organosilicone preparation is used as a pre-spray treatment of plant leaves or other plant surfaces, freshly made concentrations in the range of about 0.015 to about 2 percent by weight (wt percent) (*e. g.,* about 0.01, 0.015, 0.02, 0.025, 0.03, 0.035, 0.04, 0.045, 0.05, 0.055, 0.06, 0.065, 0.07, 0.075, 0.08, 0.085, 0.09, 0.095, 0.1, 0.2, 0.3, 0.4, 0.5, 0.6, 0.7, 0.8, 0.9, 1.0, 1.1, 1.2, 1.3, 1.4, 1.5, 1.6, 1.7, 1.8, 1.9, 2.0, 2.1, 2.2, 2.3, 2.5 wt percent) are efficacious in preparing a leaf or other plant surface for transfer of polynucleotide molecules into plant cells from a topical application on the surface. In certain embodiments of the methods and compositions provided herein, a composition that comprises a polynucleotide molecule and an organosilicone preparation comprising Silwet L-77 in the range of about 0.015 to about 2 percent by weight (wt percent) (*e. g.,* about 0.01, 0.015, 0.02, 0.025, 0.03, 0.035, 0.04, 0.045, 0.05, 0.055, 0.06, 0.065, 0.07, 0.075, 0.08, 0.085, 0.09, 0.095, 0.1, 0.2, 0.3, 0.4, 0.5, 0.6, 0.7, 0.8, 0.9, 1.0, 1.1, 1.2, 1.3, 1.4, 1.5, 1.6, 1.7, 1.8, 1.9, 2.0, 2.1, 2.2, 2.3, 2.5 wt percent) is used or provided.

In certain embodiments, any of the commercially available organosilicone preparations provided such as the following Breakthru S 321, Breakthru S 200 Cat# 67674-67-3, Breakthru OE 441 Cat#68937-55-3, Breakthru S 278 Cat #27306-78-1, Breakthru S 243, Breakthru S 233 Cat#134180-76-0, available from manufacturer Evonik Goldschmidt (Germany), Silwet® HS 429, Silwet® HS 312, Silwet® HS 508, Silwet® HS 604 (Momentive Performance Materials, Albany, New York) can be used as transfer agents in a polynucleotide composition. In certain embodiments where an organosilicone preparation is used as a pre-spray treatment of plant leaves or other surfaces, freshly made concentrations in the range of about 0.015 to about 2 percent by weight (wt percent) (*e. g.,* about 0.01, 0.015, 0.02, 0.025, 0.03, 0.035, 0.04, 0.045, 0.05, 0.055, 0.06, 0.065, 0.07, 0.075, 0.08, 0.085, 0.09, 0.095, 0.1, 0.2, 0.3, 0.4, 0.5, 0.6, 0.7, 0.8, 0.9, 1.0, 1.1, 1.2, 1.3, 1.4, 1.5, 1.6, 1.7, 1.8, 1.9, 2.0, 2.1, 2.2, 2.3, 2.5 wt percent) are efficacious in preparing a leaf or other plant surface for transfer of polynucleotide molecules into plant cells from a topical application on the surface. In certain embodiments of the methods and compositions provided herein, a composition that comprises a polynucleotide molecule and an organosilicone preparation in the range of about 0.015 to about 2 percent by weight (wt percent) (*e. g.,* about 0.01, 0.015, 0.02, 0.025, 0.03, 0.035, 0.04, 0.045, 0.05, 0.055, 0.06, 0.065, 0.07, 0.075, 0.08, 0.085, 0.09, 0.095, 0.1, 0.2, 0.3, 0.4, 0.5, 0.6, 0.7, 0.8, 0.9, 1.0, 1.1, 1.2, 1.3, 1.4, 1.5, 1.6, 1.7, 1.8, 1.9, 2.0, 2.1, 2.2, 2.3, 2.5 wt percent) is used or provided.

Organosilicone preparations used in the methods and compositions provided herein can comprise one or more effective organosilicone compounds. As used herein, the phrase "effective organosilicone compound" is used to describe any organosilicone compound that is found in an organosilicone preparation that enables a polynucleotide to enter a plant cell. In certain embodiments, an effective organosilicone compound can enable a polynucleotide to enter a plant cell in a manner permitting a polynucleotide mediated suppression of a target gene expression in the plant cell. In general, effective organosilicone compounds include, but are not limited to, compounds that can comprise: i) a trisiloxane head group that is covalently linked to, ii) an alkyl linker including, but not limited to, an n-propyl linker, that is covalently linked to, iii) a poly glycol chain, that is covalently linked to, iv) a terminal group. Trisiloxane head groups of such effective organosilicone compounds include, but are not limited to, heptamethyltrisiloxane. Alkyl linkers can include, but are not limited to, an n-propyl linker. Poly glycol chains include, but are not limited to, polyethylene glycol or polypropylene glycol. Poly glycol chains can comprise a mixture that provides an average chain length "n" of about "7.5". In certain embodiments, the average chain length "n" can vary from about 5 to about 14. Terminal groups can include, but are not limited to, alkyl groups such as a methyl group. Effective organosilicone compounds are believed to include, but are not limited to, trisiloxane ethoxylate surfactants or polyalkylene oxide modified heptamethyl trisiloxane. 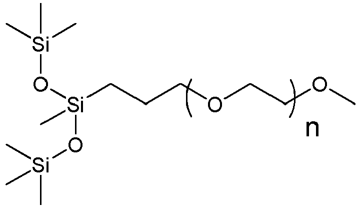 (Compound I: polyalkyleneoxide heptamethyltrisiloxane, average n=7.5)..

In certain embodiments, an organosilicone preparation that comprises an organosilicone compound comprising a trisiloxane head group is used in the methods and compositions provided herein. In certain embodiments, an organosilicone preparation that comprises an organosilicone compound comprising a heptamethyltrisiloxane head group is used in the methods and compositions provided herein. In certain embodiments, an organosilicone composition that comprises Compound I is used in the methods and compositions provided herein. In certain embodiments, an organosilicone composition that comprises Compound I is used in the methods and compositions provided herein. In certain embodiments of the methods and compositions provided herein, a composition that comprises a polynucleotide molecule and one or more effective organosilicone compound in the range of about 0.015 to about 2 percent by weight (wt percent) (*e. g.,* about 0.01, 0.015, 0.02, 0.025, 0.03, 0.035, 0.04, 0.045, 0.05, 0.055, 0.06, 0.065, 0.07, 0.075, 0.08, 0.085, 0.09, 0.095, 0.1, 0.2, 0.3, 0.4, 0.5, 0.6, 0.7, 0.8, 0.9, 1.0, 1.1, 1.2, 1.3, 1.4, 1.5, 1.6, 1.7, 1.8, 1.9, 2.0, 2.1, 2.2, 2.3, 2.5 wt percent) is used or provided.

Compositions of the present invention include but are not limited components that are one or more polynucleotides essentially identical to, or essentially complementary to an ALS gene sequence (promoter, intron, exon, 5' untranslated region, 3' untranslated region), a transfer agent that provides for the polynucleotide to enter a plant cell, a herbicide that complements the action of the polynucleotide, one or more additional herbicides that further enhance the herbicide activity of the composition or provide an additional mode of action different from the complementing herbicide, various salts and stabilizing agents that enhance the utility of the composition as an admixture of the components of the composition.

In certain aspects, methods include one or more applications of a polynucleotide composition and one or more applications of a transfer agent for conditioning of a plant to permeation by polynucleotides. When the agent for conditioning to permeation is an organosilicone composition or compound contained therein, embodiments of the polynucleotide molecules are double-stranded RNA oligonucleotides, single-stranded RNA oligonucleotides, double-stranded RNA polynucleotides, single-stranded RNA polynucleotides, double-stranded DNA oligonucleotides, single-stranded DNA oligonucleotides, double-stranded DNA polynucleotides, single-stranded DNA polynucleotides, chemically modified RNA or DNA oligonucleotides or polynucleotides or mixtures thereof.

Compositions and methods of the invention are useful for modulating the expression of an endogenous ALS gene or transgenic ALS gene (for example, U.S. Pat. No. 7,973,218; SEQ ID NO:65 comprising a soybean HRA sequence; SEQ ID NO:66 comprising a maize HRA sequence; SEQ ID NO:67 comprising an Arabidopsis HRA sequence; and SEQ ID NO:86 comprising an HRA sequence used in cottor) gene in a plant cell. In various embodiments, an ALS gene includes coding (protein-coding or translatable) sequence, non-coding (non-translatable) sequence, or both coding and non-coding sequence. Compositions can include polynucleotides and oligonucleotides designed to target multiple genes, or multiple segments of one or more genes. The target gene can include multiple consecutive segments of a target gene, multiple non-consecutive segments of a target gene, multiple alleles of a target gene, or multiple target genes from one or more species.

Provided is a method for modulating expression of an ALS gene in a plant including (a) conditioning of a plant to permeation by polynucleotides and (b) treatment of the plant with the polynucleotide molecules, wherein the polynucleotide molecules include at least one segment of 18 or more contiguous nucleotides cloned from or otherwise identified from the target ALS gene in either anti-sense or sense orientation, whereby the polynucleotide molecules permeate the interior of the plant and induce modulation of the target gene. The conditioning and polynucleotide application can be performed separately or in a single step. When the conditioning and polynucleotide application are performed in separate steps, the conditioning can precede or can follow the polynucleotide application within minutes, hours, or days. In some embodiments more than one conditioning step or more than one polynucleotide molecule application can be performed on the same plant. In embodiments of the method, the segment can be cloned or identified from (a) coding (protein-encoding), (b) non-coding (promoter and other gene related molecules), or (c) both coding and non-coding parts of the target gene. Non-coding parts include DNA, such as promoter regions or the RNA transcribed by the DNA that provide RNA regulatory molecules, including but not limited to: introns, 5' or 3' untranslated regions, and microRNAs (miRNA), *trans*-acting siRNAs, natural anti-sense siRNAs, and other small RNAs with regulatory function or RNAs having structural or enzymatic function including but not limited to: ribozymes, ribosomal RNAs, t-RNAs, aptamers, and riboswitches.

Herbicide chemical families that are known as ALS inhibiting herbicides include members of the Sulfonylureas, Imidazolinones, Triazolopyrimidines, Pyrimidinyl(thio)benzoates, and Sulfonylaminocarbonyl-triazolinones.

The following examples are included to demonstrate examples of certain preferred embodiments of the invention. It should be appreciated by those of skill in the art that the techniques disclosed in the examples that follow represent approaches the inventors have found function well in the practice of the invention, and thus can be considered to constitute examples of preferred modes for its practice.

### EXAMPLES

### Example 1. Polynucleotides related to the ALS gene sequences.

The target ALS polynucleotide molecule is represented by a large subunit and a small subunit gene that naturally occurs in the genome of *Amaranthus palmeri, Amaranthus rudis, Amaranthus chlorostachys, Amaranthus graecizans, Amaranthus hybridus, Amaranthus lividus, Amaranthus spinosus, Amaranthus thunbergii, Amaranthus viridis, Ambrosia trifida, Kochia scoparia, Abutilon theophrasti, Chenopodium album, Commelina diffusa, Conyza candensis Digitaria sanguinalis, Euphorbia heterophylla, Lolium multiflorum* and include polynucleotide molecules related to the expression of a polypeptide identified as an ALS large subunit and ALS small subunit, that include regulatory molecules, cDNAs comprising coding and noncoding regions of an ALS large subunit gene and fragments thereof and ALS small subunit gene and fragments thereof as shown in Table 1 and SEQ ID NO:1-45 and 1692-1788.

Polynucleotide molecules were extracted from these plant species by methods standard in the field, for example, total RNA was extracted using Trizol Reagent (Invitrogen Corp, Carlsbad, CA Cat. No. 15596-018), following the manufacturer's protocol or modifications thereof by those skilled in the art of polynucleotide extraction that may enhance recover or purity of the extracted RNA. Briefly, start with 1 gram of ground plant tissue for extraction. Prealiquot 10 milliliters (mL) Trizol reagent to 15 mL conical tubes. Add ground powder to tubes and shake to homogenize. Incubate the homogenized samples for 5 minutes (min) at room temperature (RT) and then add 3 mL of chloroform. Shakes tubes vigorously by hand for 15-30 seconds(sec) and incubate at RT for 3 min. Centrifuge the tubes at 7,000 revolutions per minute (rpm) for 10 min at 4 degrees C. Transfer the aqueous phase to a new 1.5 mL tube and add 1 volume of cold isopropanol. Incubate the samples for 20-30 min at RT and centrifuge at 10,000 rpm for 10 min at 4 degrees C. Wash pellet with Sigma-grade 80 percent ethanol. Remove the supernatant and briefly air-dry the pellet. Dissolve the RNA pellet in approximately 200 microliters of DEPC treated water. Heat briefly at 65 degrees C to dissolve pellet and vortex or pipet to resuspend RNA pellet. Adjust RNA concentraiton to 1-2 microgram/microliter.

DNA was extracted using EZNA SP Plant DNA Mini kit (Omega Biotek, Norcross GA, Cat#D5511) and Lysing Matrix E tubes (Q-Biogen, Cat#6914), following the manufacturer's protocol or modifications thereof by those skilled in the art of polynucleotide extraction that may enhance recover or purity of the extracted DNA. Briefly, aliquot ground tissue to a Lysing Matrix E tube on dry ice, add 800µl Buffer SP1 to each sample, homogenize in a bead beater for 35-45sec, incubate on ice for 45-60 sec, centrifuge at ≥14000 rpm for 1min at RT, add 10 microliter RNase A to the lysate, incubate at 65°C for 10min, centrifuge for 1min at RT, add 280µl Buffer SP2 and vortex to mix, incubate the samples on ice for 5min, centrifuge at ≥10,000g for 10min at RT, transfer the supernatant to a homogenizer column in a 2ml collection tube, centrifuge at 10,000g for 2min at RT, transfer the cleared lysate into a 1.5ml microfuge tube, add 1.5 volumes Buffer SP3 to the cleared lysate, vortex immediately to obtain a homogeneous mixture, transfer up to 650µl supernatant to the Hi-Bind column, centrifuge at 10,000g for 1min, repeat, apply 100µl 65°C Elution Buffer to the column, centrifuge at 10,000g for 5min at RT.

Next-generation DNA sequencers, such as the 454-FLX (Roche, Branford, CT), the SOLiD (Applied Biosystems,), and the Genome Analyzer (HiSeq2000, Illumina, San Diego, CA) were used to provide polynucleotide sequence from the DNA and RNA extracted from the plant tissues. Raw sequence data was assembled into contigs. The contig sequence was used to identify trigger molecules that can be applied to the plant to enable regulation of the gene expression.

### Example 2. Polynucleotides related to the ALS gene trigger molecules

The gene sequences and fragments of Table 1 were divided into 200 polynucleotide (200-mer) lengths with 25 polynucleotide overlapping regions (SEQ ID NO: 46-1363 and 1789-4166). These polynucleotides are tested to select the most efficacious trigger regions across the length of any target sequence. The trigger polynucleotides are constructed as sense or anti-sense ssDNA or ssRNA, dsRNA, or dsDNA, or dsDNA/RNA hybrids and combined with an organosilicone based transfer agent to provide a polynucleotide preparation. The polynucleotides are combined into sets of two to three polynucleotides per set, using 4-8 nmol of each polynucleotide. Each polynucleotide set is prepared with the transfer agent and applied to a plant or a field of plants in combination with a ALS inhibitor herbicide, or followed by a ALS inhibitor treatment one to three days after the polynucleotide application, to determine the effect on the plant's susceptibility to ALS inhibitor. The effect is measured as stunting the growth and/or killing of the plant and is measured 8-14 days after treatment with the polynucleotide set and ALS inhibitor. The most efficacious sets are identified and the individual polynucleotides are tested in the same methods as the sets are and the most efficacious single 200-mer identified. The 200-mer sequence is divided into smaller sequences of 50-70-mer regions with 10-15 polynucleotide overlapping regions and the polynucleotides tested individually. The most efficacious 50-70-mer is further divided into smaller sequences of 25-mer regions with a 12 to 13 polynucleotide overlapping region and tested for efficacy in combination with ALS inhibitor treatment. By this method it is possible to identify an oligonucleotide or several oligonucleotides that are the most efficacious trigger molecule to effect plant sensitivity to an ALS inhibitor or modulation of ALS gene expression. The modulation of ALS gene expression is determined by the detection of ALS siRNA moleclules specific to ALS gene or by an observation of a reduction in the amount of ALS RNA transcript produced relative to an untreated plant or by merely observing the anticipated phenotype of the application of the trigger with an ALS inhibiting herbicide. Detection of siRNA can be accomplished, for example, using kits such as mirVana (Ambion, Austin TX) and mirPremier (Sigma-Aldrich, St Louis, MO).

The gene sequences and fragments of Table 1 are compared and 21-mers of contiguous polynucleotides are identified that have homology across the various ALS gene sequences. The purpose is to identify trigger molecules that are useful as herbicidal molecules or in combination with an ALS inhibitor herbicide across a broad range of weed species. The sequences SEQ ID NO: 1364-1691 and 4167-4201 represent the 21-mers that are present in the ALS large subunit and small subunit genes of at least seven of the weed species of Table 1. It is contemplated that additional 21-mers can be selected from the sequences of Table 1 that are specific for a single weed species or a few weeds species within a genus or trigger molecules that are at least 18 contiguous nucleotides, at least 19 contiguous nucleotides, at least 20 contiguous nucleotides or at least 21 contiguous nucleotides in length and at least 85 percent identical to an ALS large subuintgene sequence or ALS small subunit gene sequence or a combination of both selected from the group consisting of SEQ ID NO:1-45 and 1692-1788.

By this method it is possible to identify an oligonucleotide or several oligonucleotides that are the most efficacious trigger molecule to effect plant sensitivity to an ALS inhibitor or modulation of ALS gene expression. The modulation of ALS gene expression is determined by the detection of ALS siRNA moleclules specific to ALS gene or by an observation of a reduction in the amount of ALS RNA transcript produced relative to an untreated plant. Detection of siRNA can be accomplished, for example, using kits such as mirVana (Ambion, Austin TX) and mirPremier (Sigma-Aldrich, St Louis, MO).

### Example 3. Methods related to treating plants or plant parts with a topical mixture of the trigger molecules for ALS gene expression modulation.

Single stranded or double stranded DNA or RNA fragments in sense or antisense orientation are identified and mixed with a transfer agent and other components in the composition of the invention. This composition is topically applied to plants to effect expression of the target ALS genes in the specified plant to obtain the desired effect on growth or development.

In this example, growing *Amaranthus palmeri* plants were treated with a topically applied composition for inducing modulation of a target gene in a plant including (a) an agent for conditioning of a plant to transfer of the polynucleotides and (b) polynucleotides including at least one ssDNA polynucleotide strand including at least one segment of 18-21 contiguous nucleotides of the target gene in sense (S) orientation. *Amaranthus palmeri* plants were treated with a topically applied adjuvant solution comprising a pool of ssDNA ALS2 oligonucleotides shown in Table 2 that are essentially homologous or essentially complementary to the *Amaranthus palmeri* ALS2 gene promoter sequence, 0.5% Silwet L-77 solution, 2% ammonium sulfate and 20mM sodium phosphate buffer (pH 6.8). The name of the each trigger molecule is in the left column and the sequence of the trigger molecule is shown in the right column.

**Table 2. ssDNA-ALS2 promoter trigger polynucleotides**

| Oligo name | DNA sequence | SEQ ID NO: |
|---|---|---|
| ALS_PRO_ S1 | TCTTCTCCGACTCTCACAA | 4202 |
| ALS_PRO_ S2 | TCTTCCACCCTCTCTAATG | 4203 |
| ALS_ PRO_ S3 | GGTGGAAAGATTGGAACTT | 4204 |
| ALS_PRO_ S4 | TCGTTTGTGGGTTTGTAAG | 4205 |
| ALS_PRO_ S5 | GCAATGGAAGTTTCTGCAA | 4206 |
| ALS_PRO_ S6 | AGTTCCTGTTTCAGCTCAT | 4207 |
| ALS_PRO_S7 | TGTATGTCAAGGTTTAGGTTG | 4208 |
| ALS_PRO_ S8 | GCAATAAGGTGATGGCGTG | 4209 |
| ALS_PRO_ S9 | GCGCCTCCACTATCTTCTT | 4210 |
| ALS_PRO_ S10 | GCTTTCCTCTCGCACTAAT | 4211 |
| ALS_ PRO_ S11 | CCATTTACGCTATCCCTTT | 4212 |
| ALS_ PRO_ S12 | CCCACTTCTTCTTCTTCAA | 4213 |
| ALS_PRO_ S13 | CCTAAACCTAAACCTCCTT | 4214 |
| ALS_ PRO_ S14 | TGTTCTCGTTGAAGCTCTT | 4215 |
| ALS_ PRO_ S15 | GGAAATCCATCAAGCTCTT | 4216 |
| ALS_PRO_ S16 | GGAGTTTGTATTGCCACTT | 4217 |

Approximately four-week old greenhouse grown *Amaranthus palmeri* plants (glyphosate-resistant Palmer amaranth, "R-22") were used in this assays. The plants were spray treated in a greenhouse with the ssDNA ALS2 oligonucleotide composition/0.5% Silwet L-77 solution/2% ammonium sulfate/20 mM sodium phosphate, concentration of the each oligonucleotide was approximately 16 nmol. Spray solutions were prepared the same day as spraying and applied using a track sprayer with a FLATfan nozzle 9501E at 11.4 bar at a rate of 93 g/ha (grams/hectare). The ssDNA ALS2 oligonucleotide composition was applied and followed 24 hours later by the herbicide treatment, Staple® (2-Chloro-6-{(4,6-dimethoxy-2-pyrimidinyl)thio}benzoic acid sodium salt, pyrithiobac sodium 85 percent, Dupont, Wilmington, DE) at ¼ X (27 g ai/ha (grams active ingredient per hectare) rate for the ssDNA ALS2 oligonucleotide treated plants and IX rate (108 g ai/ha) for the untreated plants, five replications per treatment and the data is presented as percentage height of the untreated control. Plant height is determined at seven days after herbicide treatment.

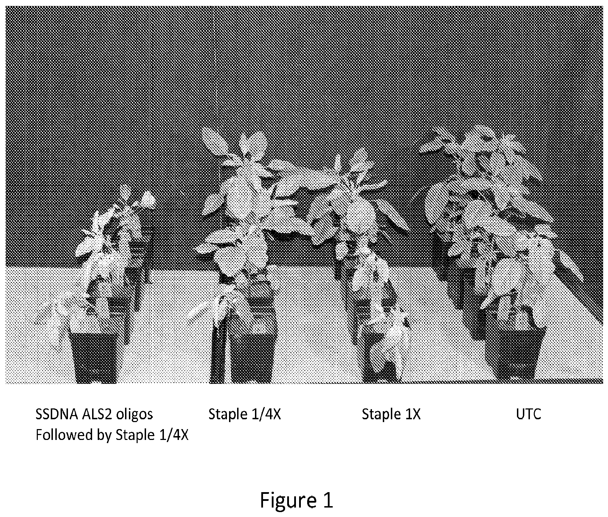

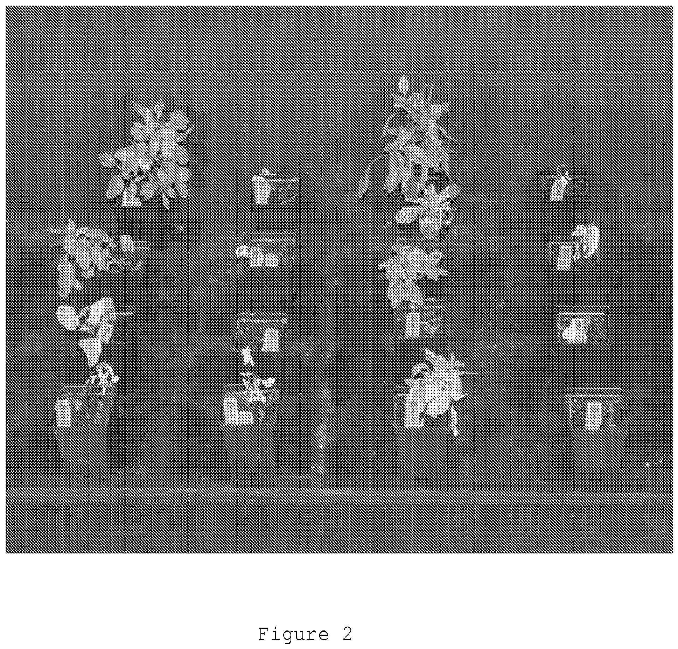

The resulting plants treated with Staple® alone at 27 g ai/ha (1/4X field rate) showed moderate growth stunting, causing between 30 percent and 35 percent growth reduction compared to the untreated control. Plants treated with Staple® at 108 g ai/ha (IX field rate) showed 40 percent growth reduction. Plants treated with ssDNA ALS2 oligonucleotides followed by Staple® at 27 g ai/ha at 1 h or 24 h showed a significant increase in growth stunting compared to Staple® alone. Thus, ssDNA ALS2 oligonucleotide treatment followed by Staple® at 1 h and 24 h caused 50 percent and 60 percent growth reduction, relative to the untreated control, while Staple® alone at 27 (1 h and 24 h) and 108 (24 h) g ai/ha caused 35 percent, 30 percent, and 40 percent growth reduction, respectively. A photograph of the treated plants is shown in Figure 1. Individual trigger molecules enhancing ALS inhibitor herbicide enhancing activity were selected by repeating the testing with the individual oligonucleotides or combinations thereof, for example, ALS_pro_S1 (SEQ ID NO:4202) was determined to be an effective trigger molecule. ALS_pro_S1 dsDNA trigger molecule was applied to ALS inhibitor resistant Palmer amaranth plants (A3, ALS inhibitor herbicide biotype) followed by ALS inhibitor herbicides IX Staple® at 108 g ai/ha or 2X Classic® (Chlorimuron Ethyl 25DF, Dupont, Willmington, DE, 2X rate is 70 g ai/ha) at + COC (crop oil concentrate) 24hr after trigger application. The results of the treatment demonstrates that the ALS_pro_S1 trigger molecule substantially improved the activity of the Staple® and Classic® herbicides on ALS inhibitor resistant plants, Figure 2.

### Example 4. Tiling test of pooled trigger molecule to ALS large subunit

Pools of trigger molecules (ALStile) were tested across the ALS large subunit gene to select for effective trigger molecules. Approximately four-week old (7.6-10.2 cm) greenhouse grown *Amaranthus palmeri* plants (Palmer amaranth A3) were used in these assays. The plants were spray treated in a greenhouse with the ALStile composition, each composition having six trigger oligonucleotides (Table 3) and a formulation comprising 1 percent Silwet L-77 solution, 2 percent ammonium sulfate and 20 mM sodium phosphate and the concentration of each oligonucleotide was approximately 4 nmol. Spray solutions were prepared the same day as spraying and applied using a track sprayer with a FLATfan nozzle 9501E at 11.4 bar at a rate of 93 g/ha (grams/hectare). The dsDNA ALStile pool oligonucleotide composition was applied and followed 24 hours later by the herbicide treatment, Staple® (2-Chloro-6-{(4,6-dimethoxy-2-pyrimidinyl)thio}benzoic acid sodium salt, pyrithiobac sodium 85 percent, Dupont, Wilmington, DE) at 108 g/ha (grams per hectare), five replications per treatment and the data is presented as a visual score of percentage injury determined fourteen days after herbicide application.

The results of these test identified three pools with herbicide enhancing activity relative to the formulation control on the A3 biotype, these were ALStile13-18 (SEQ ID NO:4218-4223), ALStile19-24 (SEQ ID NO:4224-4229) and ALStile61-66 (SEQ ID NO:4230-4235) and ALSpro_S1 (SEQ ID NO:4202, identified as an effective trigger from Example 3). Individual effective trigger molecules can be isolated from the pools and combined as necessary with other selected effective trigger molecules to enhance ALS inhibiting herbicide activity.

**Table 3. Trigger molecule tiling test for Palmer amaranth ALS large subunit gene**

| Trigger | type | Herbicide | Rep1 | Rep2 | Rep3 | Rep4 | Rep5 | avg |
|---|---|---|---|---|---|---|---|---|
| ALStile1-6 | dsDNA | Staple @ 108g/ha | 20 | 35 | 60 | 20 | 15 | 30 |
| ALStile7-12 | dsDNA | Staple @ 108g/ha | 25 | 80 | 80 | 70 | 20 | 55 |
| ALStile13-18 | dsDNA | Staple @ 108g/ha | 25 | 90 | 95 | 95 | 80 | 77 |
| ALStile19-24 | dsDNA | Staple @ 108g/ha | 15 | 80 | 98 | 95 | 95 | 76 |
| ALStile25-30 | dsDNA | Staple @ 108g/ha | 0 | 35 | 95 | 95 | 85 | 62 |
| ALStile31-36 | dsDNA | Staple @ 108g/ha | 10 | 5 | 70 | 95 | 10 | 38 |
| ALStile37-42 | dsDNA | Staple @ 108g/ha | 3 | 3 | 0 | 85 | 75 | 33 |
| ALStile43-48 | dsDNA | Staple @ 108g/ha | 5 | 8 | 50 | 10 | 0 | 14 |
| ALStile53-54 | dsDNA | Staple @ 108g/ha | 3 | 15 | 90 | 85 | 10 | 40 |
| ALStile55,57-60 | dsDNA | Staple @ 108g/ha | 3 | 15 | 98 | 99 | 15 | 46 |
| ALStile61-66 | dsDNA | Staple @ 108g/ha | 10 | 50 | 99 | 100 | 90 | 70 |
| ALStile67-72 | dsDNA | Staple @ 108g/ha | 10 | 10 | 85 | 98 | 85 | 57 |
| ALStile73-78 | dsDNA | Staple @ 108g/ha | 15 | 0 | 60 | 80 | 20 | 35 |
| ALStile79-84 | dsDNA | Staple @ 108g/ha | 15 | 10 | 15 | 80 | 30 | 30 |
| ALStile85-88 | dsDNA | Staple @ 108g/ha | 3 | 0 | 0 | 0 | 0 | 0.6 |
| ALStile89-92 | dsDNA | Staple @ 108g/ha | 0 | 0 | 0 | 0 | 3 | 0.6 |
| ALSpro_S1 | dsRNA | Staple @ 108g/ha | 100 | 100 | 100 | 100 | 100 | 100 |
| formulation | | Staple @ 108g/ha | 85 | 80 | 60 | 50 | 10 | 57 |
| Untreated | | | 0 | 0 | 0 | 0 | 0 | 0 |

### Example 5. Tiling test of pooled trigger molecules to ALS small subunit gene

Pools of trigger molecules (ALSprotile) were tested to query across the ALS small subunit gene promoter to select for effective trigger molecules. Approximately, 5.1-10.2 cm tall R-22 greenhouse grown *Amaranthus palmeri* plants (R-22) were used in these assays. The plants were spray treated in a greenhouse with the ALSprotile composition, each composition having six trigger oligonucleotides (Table 4) and a formulation comprising 1 percent Silwet L-77 solution, 2 percent ammonium sulfate and 20 mM sodium phosphate and the concentration of each oligonucleotide was approximately 4 nmol. Spray solutions were prepared the same day as spraying and applied using a track sprayer with a FLATfan nozzle 9501E at 11.4 bar at a rate of 93 g/ha (grams/hectare). The dsDNA ALSprotile pool oligonucleotide composition was applied and followed 24 hours later by the herbicide treatment, Staple® at 108 g/ha (grams per hectare), five replications per treatment and the data is presented as a visual score of percentage injury determined fourteen days after herbicide application. The results of these tests identified two pools with herbicide enhancing activity relative to the formulation control on the A3 biotype, these were ALSprotile 1-6 (SEQ ID NO:4236-4241) and ALSprotile7-12 (SEQ ID NO:4242-4247) which demonstrated 67 percent and 62 percent injury, respectfully. Individual effective trigger molecules can be isolated from the pools and combined as necessary with other selected effective trigger molecules to enhance ALS inhibiting herbicide activity.

**Table 4. Trigger molecule tiling test for Palmer amaranth ALS small subunit gene promoter**

| Trigger | type | Herbicide | Rep1 | Rep2 | Rep3 | Rep4 | Rep5 | avg |
|---|---|---|---|---|---|---|---|---|
| ALSprotile1-6 | dsDNA | Staple @ 108g/ha | 45 | 100 | 50 | 75 | 65 | 67 |
| ALStile7-12 | dsDNA | Staple @ 108g/ha | 50 | 75 | 20 | 100 | 65 | 62 |
| ALStile13-18 | dsDNA | Staple @ 108g/ha | 55 | 30 | 65 | 60 | 80 | 58 |
| ALStile19-24 | dsDNA | Staple @ 108g/ha | 30 | 40 | 75 | 60 | 70 | 55 |
| formulation | | Staple @ 108g/ha | 60 | 45 | 45 | 55 | 40 | 49 |

### Example 6. A method to control weeds in a field.

A method to control weeds in a field comprises the use of trigger polynucleotides that can modulate the expression of an ALS gene in one or more target weed plant species. An analysis of ALS gene sequences from eighteen plant species provided a collection of 21-mer polynucleotides that can be used in compositions to affect the growth or develop or sensitivity to ALS inhibitor herbicide to control multiple weed species in a field. A composition containing 1 or 2 or 3 or 4 or more of the polynucleotides of SEQ ID NO: 1364-1691 and 4167-4201 would enable broad activity of the composition against the multiple weed species that occur in a field environment.

The method includes creating a composition that comprises components that include at least one polynucleotide of SEQ ID NO: 1364-1691 and 4167-4201 or any other effective gene expression modulating polynucleotide essentially identical or essentially complementary to SEQ ID NO:1-45 and 1692-1788 or fragment thereof, a transfer agent that mobilizes the polynucleotide into a plant cell and a ALS inhibiting herbicide and optionally a polynucleotide that modulates the expression of an essential gene and optionally a co-herbicide that has a different mode of action relative to an ALS inhibitor herbicide, or a co-herbicide that has a similar mode of action of any one ALS inhibitor herbicide and is a member of a different chemical family. The polynucleotide of the composition includes a dsRNA, ssDNA or dsDNA or a combination thereof. A composition containing a polynucleotide can have a use rate of about 1 to 30 grams or more per acre depending on the size of the polynucleotide and the number of polynucleotides in the composition. The composition may include one or more additional herbicides as needed to provide effective multi-species weed control. For example, a composition comprising an ALS gene trigger oligonucleotide, the composition further including a co-herbicide but not limited to acetochlor, acifluorfen, acifluorfen-sodium, aclonifen, acrolein, alachlor, alloxydim, allyl alcohol, ametryn, amicarbazone, amidosulfuron, aminopyralid, amitrole, ammonium sulfamate, anilofos, asulam, atraton, atrazine, azimsulfuron, BCPC, beflubutamid, benazolin, benfluralin, benfuresate, bensulfuron, bensulfuron-methyl, bensulide, bentazone, benzfendizone, benzobicyclon, benzofenap, bifenox, bilanafos, bispyribac, bispyribac-sodium, borax, bromacil, bromobutide, bromoxynil, butachlor, butafenacil, butamifos, butralin, butroxydim, butylate, cacodylic acid, calcium chlorate, cafenstrole, carbetamide, carfentrazone, carfentrazone-ethyl, CDEA, CEPC, chlorflurenol, chlorflurenol-methyl, chloridazon, chlorimuron, chlorimuron-ethyl, chloroacetic acid, chlorotoluron, chlorpropham, chlorsulfuron, chlorthal, chlorthal-dimethyl, cinidon-ethyl, cinmethylin, cinosulfuron, cisanilide, clethodim, clodinafop, clodinafop-propargyl, clomazone, clomeprop, clopyralid, cloransulam, cloransulam-methyl, CMA, 4-CPB, CPMF, 4-CPP, CPPC, cresol, cumyluron, cyanamide, cyanazine, cycloate, cyclosulfamuron, cycloxydim, cyhalofop, cyhalofop-butyl, 2,4-D, 3,4-DA, daimuron, dalapon, dazomet, 2,4-DB, 3,4-DB, 2,4-DEB, desmedipham, dicamba, dichlobenil, ortho-dichlorobenzene, para-dichlorobenzene, dichlorprop, dichlorprop-P, diclofop, diclofop-methyl, diclosulam, difenzoquat, difenzoquat metilsulfate, diflufenican, diflufenzopyr, dimefuron, dimepiperate, dimethachlor, dimethametryn, dimethenamid, dimethenamid-P, dimethipin, dimethylarsinic acid, dinitramine, dinoterb, diphenamid, diquat, diquat dibromide, dithiopyr, diuron, DNOC, 3,4-DP, DSMA, EBEP, endothal, EPTC, esprocarb, ethalfluralin, ethametsulfuron, ethametsulfuron-methyl, ethofumesate, ethoxyfen, ethoxysulfuron, etobenzanid, fenoxaprop-P, fenoxaprop-P-ethyl, fentrazamide, ferrous sulfate, flamprop-M, flazasulfuron, florasulam, fluazifop, fluazifop-butyl, fluazifop-P, fluazifop-P-butyl, flucarbazone, flucarbazone-sodium, flucetosulfuron, fluchloralin, flufenacet, flufenpyr, flufenpyr-ethyl, flumetsulam, flumiclorac, flumiclorac-pentyl, flumioxazin, fluometuron, fluoroglycofen, fluoroglycofen-ethyl, flupropanate, flupyrsulfuron, flupyrsulfuron-methyl-sodium, flurenol, fluridone, fluorochloridone, fluoroxypyr, flurtamone, fluthiacet, fluthiacet-methyl, fomesafen, foramsulfuron, fosamine, glufosinate, glufosinate-ammonium, halosulfuron, halosulfuron-methyl, haloxyfop, haloxyfop-P, HC-252, hexazinone, imazamethabenz, imazamethabenz-methyl, imazamox, imazapic, imazapyr, imazaquin, imazethapyr, imazosulfuron, indanofan, iodomethane, iodosulfuron, iodosulfuron-methyl-sodium, ioxynil, isoproturon, isouron, isoxaben, isoxachlortole, isoxaflutole, karbutilate, lactofen, lenacil, linuron, MAA, MAMA, MCPA, MCPA-thioethyl, MCPB, mecoprop, mecoprop-P, mefenacet, mefluidide, mesosulfuron, mesosulfuron-methyl, mesotrione, metam, metamifop, metamitron, metazachlor, methabenzthiazuron, methylarsonic acid, methyldymron, methyl isothiocyanate, metobenzuron, metolachlor, S-metolachlor, metosulam, metoxuron, metribuzin, metsulfuron, metsulfuron-methyl, MK-66, molinate, monolinuron, MSMA, naproanilide, napropamide, naptalam, neburon, nicosulfuron, nonanoic acid, norflurazon, oleic acid (fatty acids), orbencarb, orthosulfamuron, oryzalin, oxadiargyl, oxadiazon, oxasulfuron, oxaziclomefone, oxyfluorfen, paraquat, paraquat dichloride, pebulate, pendimethalin, penoxsulam, pentachlorophenol, pentanochlor, pentoxazone, pethoxamid, petrolium oils, phenmedipham, phenmedipham-ethyl, picloram, picolinafen, pinoxaden, piperophos, potassium arsenite, potassium azide, pretilachlor, primisulfuron, primisulfuron-methyl, prodiamine, profluazol, profoxydim, prometon, prometryn, propachlor, propanil, propaquizafop, propazine, propham, propisochlor, propoxycarbazone, propoxycarbazone-sodium, propyzamide, prosulfocarb, prosulfuron, pyraclonil, pyraflufen, pyraflufen-ethyl, pyrazolynate, pyrazosulfuron, pyrazosulfuron-ethyl, pyrazoxyfen, pyribenzoxim, pyributicarb, pyridafol, pyridate, pyriftalid, pyriminobac, pyriminobac-methyl, pyrimisulfan, pyrithiobac, pyrithiobac-sodium, quinclorac, quinmerac, quinoclamine, quizalofop, quizalofop-P, rimsulfuron, sethoxydim, siduron, simazine, simetryn, SMA, sodium arsenite, sodium azide, sodium chlorate, sulcotrione, sulfentrazone, sulfometuron, sulfometuron-methyl, sulfosate, sulfosulfuron, sulfuric acid, tar oils, 2,3,6-TBA, TCA, TCA-sodium, tebuthiuron, tepraloxydim, terbacil, terbumeton, terbuthylazine, terbutryn, thenylchlor, thiazopyr, thifensulfuron, thifensulfuron-methyl, thiobencarb, tiocarbazil, topramezone, tralkoxydim, tri-allate, triasulfuron, triaziflam, tribenuron, tribenuron-methyl, tricamba, triclopyr, trietazine, trifloxysulfuron, trifloxysulfuron-sodium, trifluralin, triflusulfuron, triflusulfuron-methyl, trihydroxytriazine, tritosulfuron, [3-[2-chloro-4-fluoro-5-(-methyl-6-trifluoromethyl-2,4-dioxo-,2,3,4-t- etrahydropyrimidin-3-yl)phenoxy]-2-pyridyloxy]acetic acid ethyl ester (CAS RN 353292-3-6), 4-[(4,5-dihydro-3-methoxy-4-methyl-5-oxo)-H-,2,4-triazol--ylcarbonyl-sulfamoyl]-5-methylthiophene-3-carboxylic acid (BAY636), BAY747 (CAS RN 33504-84-2), topramezone (CAS RN 2063-68-8), 4-hydroxy-3-[[2-[(2-methoxyethoxy)methyl]-6-(trifluoro-methyl)-3-pyridi- nyl]carbonyl]-bicyclo[3.2.]oct-3-en-2-one (CAS RN 35200-68-5), and 4-hydroxy-3-[[2-(3-methoxypropyl)-6-(difluoromethyl)-3-pyridinyl]carbon-yl]-bicyclo[3.2.]oct-3-en-2-one.

A field of crop plants in need of weed plant control is treated by spray application of the composition. The composition can be provided as a tank mix, a sequential treatment of components (generally the polynucleotide followed by the herbicide), a simultaneous treatment or mixing of one or more of the components of the composition from separate containers. Treatment of the field can occur as often as needed to provide weed control and the components of the composition can be adjusted to target specific weed species or weed families.

### Example 7. Herbicidal Compositions comprising pesticidal agents

A method of controlling weeds and plant pest and pathogens in a field of ALS inhibitor tolerant crop plants is provided, wherein the method comprises applying a composition comprising an ALS trigger polynucleotide, an ALS inhibitor herbicide composition and an admixture of a pest control agent. For example, the admixture comprises insecticides, fungicides, nematocides, bactericides, acaricides, growth regulators, chemosterilants, semiochemicals, repellents, attractants, pheromones, feeding stimulants or other biologically active compounds or biological agents, such as, microorganisms.

For example, the admixture comprises a fungicide compound for use on an ALS herbicide tolerant crop plant to prevent or control plant disease caused by a plant fungal pathogen, The fungicide compound of the admixture may be a systemic or contact fungicide or mixtures of each. More particularly the fungicide compound includes, but is not limited to members of the chemical groups strobilurins, triazoles, chloronitriles, carboxamides and mixtures thereof. The composition may additional have an admixture comprises an insecticidal compound or agent.

Agricultural chemicals are provided in containers suitable for safe storage, transportation and distribution, stability of the chemical compositions, mixing with solvents and instructions for use. A container of the compositions of the present invention may include mixture of a trigger oligonucleotide + ALS inhibitor herbicide + fungicide compound, or a mixture of a trigger oligonucleotide + ALS inhibitor herbicide compound and an insecticide compound, or a trigger oligonucleotide + a ALS inhibitor herbicide compound and a fungicide compound and an insecticide compound (for example, lambda-cyhalothrin, Warrier®). The container may further provide instructions on the effective use of the mixture. Containers of the present invention can be of any material that is suitable for the storage of the chemical mixture. Containers can be of any material that is suitable for the shipment of the chemical mixture. The material can be of cardboard, plastic, metal, or a composite of these materials. The container can have a volume of 0.5 liter, 1 liter, 2 liter, 3-5 liter, 5-10 liter, 10-20 liter, 20-50 liter or more depending upon the need. A tank mix of a trigger oligonucleotide + ALS inhibitor herbicide compound and a fungicide compound is provided, methods of application to the crop to achieve an effective dose of each compound are known to those skilled in the art and can be refined and further developed depending on the crop, weather conditions, and application equipment used.

Insecticides, fungicides, nematocides, bactericides, acaricides, growth regulators, chemosterilants, semiochemicals, repellents, attractants, pheromones, feeding stimulants or other biologically active compounds can be added to the trigger oligonucleotide to form a multi-component pesticide giving an even broader spectrum of agricultural protection. Examples of such agricultural protectants with which compounds of this invention can be formulated are: insecticides such as abamectin, acephate, azinphos-methyl, bifenthrin, buprofezin, carbofuran, chlorfenapyr, chlorpyrifos, chlorpyrifos-methyl, cyfluthrin, beta-cyfluthrin, cyhalothrin, lambda-cyhalothrin, deltamethrin, diafenthiuron, diazinon, diflubenzuron, dimethoate, esfenvalerate, fenoxycarb, fenpropathrin, fenvalerate, fipronil, flucythrinate, tau-fluvalinate, fonophos, imidacloprid, isofenphos, malathion, metaldehyde, methamidophos, methidathion, methomyl, methoprene, methoxychlor, methyl 7-chloro-2,5-dihydro-2-[[N-(methoxycarbonyl)-N-[4-(trifluoromethoxy)phenyl ]amino]carbonyl]indeno[1,2-e][1,3,4]oxadiazine-4a(3H)-carboxylate (DPX-JW062), monocrotophos, oxamyl, parathion, parathion-methyl, permethrin, phorate, phosalone, phosmet, phosphamidon, pirimicarb, profenofos, rotenone, sulprofos, tebufenozide, tefluthrin, terbufos, tetrachlorvinphos, thiodicarb, tralomethrin, trichlorfon and triflumuron; most preferably a glyphosate compound is formulated with a fungicide compound or combinations of fungicides, such as azoxystrobin, benomyl, blasticidin-S, Bordeaux mixture (tribasic copper sulfate), bromuconazole, captafol, captan, carbendazim, chloroneb, chlorothalonil, copper oxychloride, copper salts, cymoxanil, cyproconazole, cyprodinil (CGA 219417), diclomezine, dicloran, difenoconazole, dimethomorph, diniconazole, diniconazole-M, dodine, edifenphos, epoxiconazole (BAS 480F), famoxadone, fenarimol, fenbuconazole, fenpiclonil, fenpropidin, fenpropimorph, fluazinam, fluquinconazole, flusilazole, flutolanil, flutriafol, folpet, fosetyl-aluminum, furalaxyl, hexaconazole, ipconazole, iprobenfos, iprodione, isoprothiolane, kasugamycin, kresoxim-methyl, mancozeb, maneb, mepronil, metalaxyl, metconazole, S-methyl 7-benzothiazolecarbothioate (CGA 245704), myclobutanil, neo-asozin (ferric methanearsonate), oxadixyl, penconazole, pencycuron, probenazole, prochloraz, propiconazole, pyrifenox, pyroquilon, quinoxyfen, spiroxamine (KWG4168), sulfur, tebuconazole, tetraconazole, thiabendazole, thiophanate-methyl, thiram, triadimefon, triadimenol, tricyclazole, trifloxystrobin, triticonazole, validamycin and vinclozolin; combinations of fungicides are common for example, cyproconazole and azoxystrobin, difenoconazole, and metalaxyl-M, fludioxonil and metalaxyl-M, mancozeb and metalaxyl-M, copper hydroxide and metalaxyl-M, cyprodinil and fludioxonil, cyproconazole and propiconazole; commercially available fungicide formulations for control of Asian soybean rust disease include, but are not limited to Quadris® (Syngenta Corp), Bravo® (Syngenta Corp), Echo 720® (Sipcam Agro Inc), Headline® 2.09EC (BASF Corp), Tilt® 3.6EC (Syngenta Corp), PropiMax™ 3.6EC (Dow AgroSciences), Bumper® 41.8EC (MakhteshimAgan), Folicur® 3.6F (Bayer CropScience), Laredo® 25EC (Dow AgroSciences), Laredo™ 25EW (Dow AgroSciences), Stratego® 2.08F (Bayer Corp), Domark™ 125SL (Sipcam Agro USA), and Pristine®38%WDG (BASF Corp) these can be combined with compositions as described in the present invention to provide enhanced protection from soybean rust disease; nematocides such as aldoxycarb and fenamiphos; bactericides such as streptomycin; acaricides such as amitraz, chinomethionat, chlorobenzilate, cyhexatin, dicofol, dienochlor, etoxazole, fenazaquin, fenbutatin oxide, fenpropathrin, fenpyroximate, hexythiazox, propargite, pyridaben and tebufenpyrad; and biological agents such as *Bacillus thuringiensis, Bacillus thuringiensis* delta endotoxin, baculovirus, and entomopathogenic bacteria, virus and fungi.

**Table 1. Acetolactate synthase gene cDNA and gDNA contig sequences**

| SEQ ID NO | SPECIES | TYPE | LENGTH |
|---|---|---|---|
| 1 | Amaranthus chlorostachys | cDNAContig | 534 |
| 2 | Amaranthus chlorostachys | cDNAContig | 500 |
| 3 | Amaranthus graecizans | cDNAContig | 2267 |
| 4 | Amaranthus hybridus | cDNAContig | 2572 |
| 5 | Amaranthus lividus | cDNAContig | 2572 |
| 6 | Amaranthus palmeri | cDNAContig | 2348 |
| 7 | Amaranthus palmeri | gDNAContig | 6061 |
| 8 | Amaranthus palmeri | gDNAContig | 5410 |
| 9 | Amaranthus rudis | cDNAContig | 3817 |
| 10 | Amaranthus rudis | cDNAContig | 2404 |
| 11 | Amaranthus rudis | cDNAContig | 1364 |
| 12 | Amaranthus rudis | gDNAContig | 2528 |
| 13 | Amaranthus rudis | gDNAContig | 1737 |
| 14 | Amaranthus rudis | gDNAContig | 1719 |
| 15 | Amaranthus rudis | gDNAContig | 725 |
| 16 | Amaranthus rudis | gDNAContig | 451 |
| 17 | Amaranthus rudis | gDNAContig | 375 |
| 18 | Amaranthus spinosus | cDNAContig | 2302 |
| 19 | Amaranthus thunbergii | cDNAContig | 2334 |
| 20 | Amaranthus viridis | cDNAContig | 2360 |
| 21 | Amaranthus viridis | cDNAContig | 2357 |
| 22 | Ambrosia trifida | cDNAContig | 2075 |
| 23 | Ambrosia trifida | gDNAContig | 2540 |
| 24 | Ambrosia trifida | gDNAContig | 2141 |
| 25 | Ambrosia trifida | gDNAContig | 687 |
| 26 | Ambrosia trifida | gDNAContig | 322 |
| 27 | Ambrosia trifida | gDNAContig | 214 |
| 28 | Chenopodium album | cDNAContig | 2572 |
| 29 | Chenopodium album | cDNAContig | 2103 |
| 30 | Conyza canadensis | cDNAContig | 2379 |
| 31 | Conyza canadensis | gDNAContig | 5989 |
| 32 | Conyza canadensis | gDNAContig | 5989 |
| 33 | Euphorbia heterophylla | cDNAContig | 2053 |
| 34 | Euphorbia heterophylla | gDNAContig | 4912 |
| 35 | Euphorbia heterophylla | gDNAContig | 3185 |
| 36 | Commelina diffusa | cDNAContig | 363 |
| 37 | Digitaria sanguinalis | cDNAContig | 1868 |
| 38 | Digitaria sanguinalis | gDNAContig | 5667 |
| 39 | Kochia scoparia | cDNAContig | 2001 |
| 40 | Kochia scoparia | cDNAContig | 2001 |
| 41 | Kochia scoparia | gDNAContig | 6022 |
| 42 | Kochia scoparia | gDNAContig | 2514 |
| 43 | Lolium multiflorum | cDNAContig | 2152 |
| 44 | Lolium multiflorum | gDNAContig | 14102 |
| 45 | Lolium multiflorum | gDNAContig | 206 |
| 1692 | Abutilon theophrasti | cDNAContig | 1599 |
| 1693 | Amaranthus graecizans | cDNAContig | 714 |
| 1694 | Amaranthus hybridus | cDNAContig | 632 |
| 1695 | Amaranthus hybridus | cDNAContig | 495 |
| 1696 | Amaranthus lividus | cDNAContig | 1326 |
| 1697 | Amaranthus lividus | cDNAContig | 621 |
| 1698 | Amaranthus lividus | cDNAContig | 509 |
| 1699 | Amaranthus palmeri | cDNAContig | 1678 |
| 1700 | Amaranthus palmeri | cDNAContig | 1483 |
| 1701 | Amaranthus palmeri | cDNAContig | 1480 |
| 1702 | Amaranthus palmeri | gDNAContig | 9758 |
| 1703 | Amaranthus palmeri | gDNAContig | 7941 |
| 1704 | Amaranthus palmeri | gDNAContig | 5448 |
| 1705 | Amaranthus palmeri | gDNAContig | 5396 |
| 1706 | Amaranthus palmeri | gDNAContig | 5223 |
| 1707 | Amaranthus palmeri | gDNAContig | 4591 |
| 1708 | Amaranthus palmeri | gDNAContig | 4516 |
| 1709 | Amaranthus rudis | cDNAContig | 1767 |
| 1710 | Amaranthus rudis | cDNAContig | 1560 |
| 1711 | Amaranthus rudis | cDNAContig | 341 |
| 1712 | Amaranthus rudis | gDNAContig | 7847 |
| 1713 | Amaranthus rudis | gDNAContig | 4818 |
| 1714 | Amaranthus rudis | gDNAContig | 1687 |
| 1715 | Amaranthus rudis | gDNAContig | 1648 |
| 1716 | Amaranthus rudis | gDNAContig | 1125 |
| 1717 | Amaranthus rudis | gDNAContig | 1029 |
| 1718 | Amaranthus rudis | gDNAContig | 713 |
| 1719 | Amaranthus spinosus | cDNAContig | 1392 |
| 1720 | Amaranthus spinosus | cDNAContig | 579 |
| 1721 | Amaranthus spinosus | cDNAContig | 497 |
| 1722 | Amaranthus thunbergii | cDNAContig | 495 |
| 1723 | Amaranthus viridis | cDNAContig | 1729 |
| 1724 | Amaranthus viridis | cDNAContig | 1726 |
| 1725 | Amaranthus viridis | cDNAContig | 1565 |
| 1726 | Amaranthus viridis | cDNAContig | 1562 |
| 1727 | Amaranthus viridis | cDNAContig | 1441 |
| 1728 | Amaranthus viridis | cDNAContig | 1257 |
| 1729 | Amaranthus viridis | cDNAContig | 1254 |
| 1730 | Amaranthus viridis | cDNAContig | 1090 |
| 1731 | Ambrosia trifida | cDNAContig | 1616 |
| 1732 | Ambrosia trifida | cDNAContig | 1597 |
| 1733 | Ambrosia trifida | gDNAContig | 3632 |
| 1734 | Ambrosia trifida | gDNAContig | 1966 |
| 1735 | Ambrosia trifida | gDNAContig | 1865 |
| 1736 | Ambrosia trifida | gDNAContig | 1079 |
| 1737 | Ambrosia trifida | gDNAContig | 990 |
| 1738 | Ambrosia trifida | gDNAContig | 984 |
| 1739 | Ambrosia trifida | gDNAContig | 804 |
| 1740 | Ambrosia trifida | gDNAContig | 409 |
| 1741 | Conyza canadensis | cDNAContig | 1705 |
| 1742 | Conyza canadensis | cDNAContig | 498 |
| 1743 | Conyza canadensis | gDNAContig | 8066 |
| 1744 | Conyza canadensis | gDNAContig | 7484 |
| 1745 | Conyza canadensis | gDNAContig | 7382 |
| 1746 | Conyza canadensis | gDNAContig | 6631 |
| 1747 | Conyza canadensis | gDNAContig | 5498 |
| 1748 | Conyza canadensis | gDNAContig | 1105 |
| 1749 | Conyza canadensis | gDNAContig | 246 |
| 1750 | Euphorbia heterophylla | cDNAContig | 598 |
| 1751 | Euphorbia heterophylla | cDNAContig | 598 |
| 1752 | Euphorbia heterophylla | cDNAContig | 595 |
| 1753 | Euphorbia heterophylla | gDNAContig | 5587 |
| 1754 | Euphorbia heterophylla | gDNAContig | 4073 |
| 1755 | Euphorbia heterophylla | gDNAContig | 2403 |
| 1756 | Euphorbia heterophylla | gDNAContig | 1780 |
| 1757 | Euphorbia heterophylla | gDNAContig | 666 |
| 1758 | Euphorbia heterophylla | gDNAContig | 351 |
| 1759 | Euphorbia heterophylla | gDNAContig | 318 |
| 1760 | Commelina diffusa | cDNAContig | 591 |
| 1761 | Commelina diffusa | gDNAContig | 760 |
| 1762 | Commelina diffusa | gDNAContig | 374 |
| 1763 | Commelina diffusa | gDNAContig | 310 |
| 1764 | Commelina diffusa | gDNAContig | 288 |
| 1765 | Commelina diffusa | gDNAContig | 255 |
| 1766 | Commelina diffusa | gDNAContig | 210 |
| 1767 | Digitaria sanguinalis | cDNAContig | 569 |
| 1768 | Digitaria sanguinalis | gDNAContig | 5819 |
| 1769 | Digitaria sanguinalis | gDNAContig | 5263 |
| 1770 | Digitaria sanguinalis | gDNAContig | 4483 |
| 1771 | Digitaria sanguinalis | gDNAContig | 3928 |
| 1772 | Digitaria sanguinalis | gDNAContig | 579 |
| 1773 | Digitaria sanguinalis | gDNAContig | 402 |
| 1774 | Digitaria sanguinalis | gDNAContig | 227 |
| 1775 | Kochia scoparia | gDNAContig | 12412 |
| 1776 | Kochia scoparia | gDNAContig | 7214 |
| 1777 | Kochia scoparia | gDNAContig | 5508 |
| 1778 | Kochia scoparia | gDNAContig | 2741 |
| 1779 | Lolium multiflorum | gDNAContig | 1203 |
| 1780 | Lolium multiflorum | gDNAContig | 1074 |
| 1781 | Lolium multiflorum | gDNAContig | 838 |
| 1782 | Lolium multiflorum | gDNAContig | 651 |
| 1783 | Lolium multiflorum | gDNAContig | 487 |
| 1784 | Lolium multiflorum | gDNAContig | 475 |
| 1785 | Lolium multiflorum | gDNAContig | 373 |
| 1786 | Lolium multiflorum | gDNAContig | 343 |
| 1787 | Lolium multiflorum | gDNAContig | 332 |
| 1788 | Lolium multiflorum | gDNAContig | 285 |

## Claims

1. A method of plant control comprising: topically applying to a surface of a plant a composition comprising a double-stranded RNA (dsRNA) polynucleotide and a transfer agent, wherein said dsRNA polynucleotide is identical or complementary to at least 18 contiguous nucleotides of an RNA sequence of an acetolactate synthase (ALS) gene sequence, wherein said ALS gene sequence is selected from the group consisting of SEQ ID NOs:2, 15, 25-27, 30-33, 35, 36, 39-45, 1692-1697, 1699, 1705, 1707, 1709-1712, 1714-1721, 1727-1742, 1744-1774, and 1776-1788, wherein said transfer agent conditions said surface of said plant for permeation by said dsRNA polynucleotide, and whereby said plant's growth, development, or reproductive ability is reduced or said plant is more sensitive to an ALS inhibitor herbicide, relative to an untreated plant.

2. The method as claimed in claim 1, wherein
(i) said transfer agent is an organosilicone surfactant composition or an organosilicone compound contained therein;
(ii) said plant is selected from the group consisting of *Amaranthus palmeri*, *Amaranthus rudis, Amaranthus chlorostachys, Amaranthus graecizans, Amaranthus hybridus*, *Amaranthus lividus*, *Amaranthus spinosus, Amaranthus thunbergii, Amaranthus viridis, Ambrosia trifida, Kochia scoparia, Abutilon theophrasti*, *Chenopodium album, Commelina diffusa, Conyza canadensis Digitaria sanguinalis*, *Euphorbia heterophylla*, and *Lolium multiflorum; or*
(iii) said composition further comprises
(a) said ALS inhibitor herbicide; or
(b) one or more herbicides different from said ALS inhibitor herbicide.

3. The method as claimed in claim 1, wherein
said dsRNA polynucleotide is at least 19 contiguous nucleotides in length, or said composition comprises any combination of two or more of said dsRNA polynucleotides.

4. A composition for topical application to a surface of a plant comprising a dsRNA polynucleotide and a transfer agent, wherein said dsRNA polynucleotide is identical or complementary to at least 18 contiguous nucleotides of an RNA sequence of an ALS gene sequence, wherein said ALS gene sequence is selected from the group consisting of SEQ ID NOs:2, 15, 25-27, 30-33, 35, 36, 39-45, 1692-1697, 1699, 1705, 1707, 1709-1712, 1714-1721, 1727-1742, 1744-1774, and 1776-1788, wherein said transfer agent conditions said surface of said plant for permeation by said dsRNA polynucleotide, and whereby said plant treated with said composition has its growth, development, or reproductive ability suppressed or delayed or said plant is more sensitive to an ALS inhibitor herbicide as a result of said composition comprising said dsRNA polynucleotide, relative to an untreated plant.

5. The composition of claim 4, wherein
(i) said transfer agent is an organosilicone preparation;
(ii) said dsRNA polynucleotide is at least 19 contiguous nucleotides in length;
(iii) said dsRNA polynucleotide is selected from the group consisting of SEQ ID NOs:48-53, 62, 63, 66-73, 76, 77, 84, 85, 90, 91, 94-97, 100, 101, 110, 111, 114-117, 120, 121, 124, 125, 138, 139, 142-145, 148-171, 186-189, 192, 193, 196-239, 250, 251, 254-256, 257, 260, 261, 264-279, 288, 289, 294, 295, 306, 307, 310-339, 352-359, 362-365, 378-385, 388-393, 396-419, 428, 429, 432, 433, 436, 437, 440, 441, 450, 451, 454-457, 460, 461, 464-467, 476, 477, 480, 481, 486, 487, 490-493, 502, 503, 506-509, 512, 513, 516-519, 528, 529, 532-535, 538, 539, 542-559, 564-581, 584-609, 612-631, 634-637, 640-659, 662-665, 668-919, 922-1363, 1789-1840, 1843-1916, 1925-2024, 2027-2040, 2043-2060, 2063-2102, 2105-2270, 2273-2286, 2289-2404, 2407-2640, 2647-2662, 2665-2678, 2681-2696, 2699-2956, 2959-3946, and 3949-4166;
(iv) said dsRNA polynucleotide is selected from the group consisting of SEQ ID NOs:1364-1691 and 4167-4201; or
(v) said composition further comprises
(a) said ALS inhibitor herbicide;
(b) said ALS inhibitor molecule selected from the group consisting of amidosulfuron, azimsulfuron, bensulfuron-methyl, chlorimuron-ethyl, chlorsulfuron, cinosulfuron, cyclosulfamuron, ethametsulfuron-methyl, ethoxysulfuron, flazasulfuron, flupyrsulfuron-methyl-Na, foramsulfuron, halosulfuron-methyl, imazosulfuron, iodosulfuron, metsulfuron-methyl, nicosulfuron, oxasulfuron, primisulfuron-methyl, prosulfuron, pyrazosulfuron-ethyl, rimsulfuron, sulfometuron-methyl, sulfosulfuron, thifensulfuron-methyl, triasulfuron, tribenuron-methyl, trifloxysulfuron, triflusulfuron-methyl, tritosulfuron, imazapic, imazamethabenz-methyl, imazamox, imazapyr, imazaquin, imazethapyr, cloransulam-methyl, diclosulam, florasulam, flumetsulam, metosulam, bispyribac-Na, pyribenzoxim, pyriftalid, pyrithiobac-Na, pyriminobac-methyl, flucarbazone-Na, and procarbazone-Na; or
(c) a co-herbicide.

6. A method of reducing expression of an ALS gene in a plant comprising: topically applying to a surface of said plant a composition comprising a dsRNA polynucleotide and a transfer agent, wherein said dsRNA polynucleotide is identical or complementary to at least 18 contiguous nucleotides of an RNA sequence of an ALS gene sequence, wherein said ALS gene sequence is selected from the group consisting of SEQ ID NOs:2, 15, 25-27, 30-33, 35, 36, 39-45, 1692-1697, 1699, 1705, 1707, 1709-1712, 1714-1721, 1727-1742, 1744-1774, and 1776-1788, wherein said transfer agent conditions said surface of said plant for permeation by said dsRNA polynucleotide, whereby said expression of said ALS gene is reduced, relative to an untreated plant.

7. The method as claimed in claim 6, wherein
(i) said transfer agent is an organosilicone preparation; or
(ii) said dsRNA polynucleotide is at least 19 contiguous nucleotides in length.

8. A method of identifying dsRNA polynucleotides useful in modulating ALS gene expression when topically applied to a surface of a plant comprising: a) providing a plurality of dsRNA polynucleotides that comprise a region identical or complementary to at least 18 contiguous nucleotides of an RNA sequence of an ALS gene sequence selected from the group consisting of SEQ ID NOs:2, 15, 25-27, 30-33, 35, 36, 39-45, 1692-1697, 1699, 1705, 1707, 1709-1712, 1714-1721, 1727-1742, 1744-1774, and 1776-1788; b) topically applying to said surface of said plant a composition comprising one or more of said dsRNA polynucleotides and a transfer agent; and c) analyzing said plant, or a plant extract thereof, for modulation of ALS gene expression, wherein said transfer agent conditions said surface of said plant for permeation by said one or more of said dsRNA polynucleotides, and whereby said plant treated with said composition has its growth, development, or reproductive ability suppressed or delayed or said plant is more sensitive to an ALS inhibitor herbicide as a result of said composition, relative to an untreated plant.

9. The method as claimed in claim 8, wherein
(i) said plant is selected from the group consisting of *Amaranthus palmeri*, *Amaranthus rudis, Amaranthus chlorostachys, Amaranthus graecizans, Amaranthus hybridus*, *Amaranthus lividus*, *Amaranthus spinosus, Amaranthus thunbergii, Amaranthus viridis*, *Ambrosia trifida*, *Kochia scoparia, Abutilon theophrasti*, *Chenopodium album, Commelina diffusa, Conyza canadensis Digitaria sanguinalis*, *Euphorbia heterophylla*, and *Lolium multiflorum;* or
(ii) said ALS gene expression is reduced relative to a plant not treated with said composition; or
(iii) said transfer agent is an organosilicone preparation.

10. An agricultural chemical composition for topical application to a surface of a plant comprising an admixture of a dsRNA polynucleotide, glyphosate, and a co-herbicide, wherein said dsRNA polynucleotide is identical or complementary to at least 18 contiguous nucleotides of an RNA sequence of an ALS gene sequence, wherein said ALS gene sequence is selected from the group consisting of SEQ ID NOs:2, 15, 25-27, 30-33, 35, 36, 39-45, 1692-1697, 1699, 1705, 1707, 1709-1712, 1714-1721, 1727-1742, 1744-1774, and 1776-1788, and whereby said plant treated with said composition has its growth, development, or reproductive ability suppressed or delayed or said plant is more sensitive to an ALS inhibitor herbicide as a result of said composition comprising said dsRNA polynucleotide, relative to an untreated plant.

11. The agricultural chemical composition of claim 10, wherein said co-herbicide is selected from the group consisting of amide herbicides, arsenical herbicides, benzothiazole herbicides, benzoylcyclohexanedione herbicides, benzofuranyl alkylsulfonate herbicides, carbamate herbicides, cyclohexene oxime herbicides, cyclopropylisoxazole herbicides, dicarboximide herbicides, dinitroaniline herbicides, dinitrophenol herbicides, diphenyl ether herbicides, dithiocarbamate herbicides, halogenated aliphatic herbicides, imidazolinone herbicides, inorganic herbicides, nitrile herbicides, organophosphorus herbicides, oxadiazolone herbicides, oxazole herbicides, phenoxy herbicides, phenylenediamine herbicides, pyrazole herbicides, pyridazine herbicides, pyridazinone herbicides, pyridine herbicides, pyrimidinediamine herbicides, pyrimidinyloxybenzylamine herbicides, quaternary ammonium herbicides, thiocarbamate herbicides, thiocarbonate herbicides, thiourea herbicides, triazine herbicides, triazinone herbicides, triazole herbicides, triazolone herbicides, triazolopyrimidine herbicides, uracil herbicides, and urea herbicides.

12. An agricultural chemical composition for topical application to a surface of a plant comprising an admixture of a dsRNA polynucleotide, glyphosate, and a pesticide, wherein said dsRNA polynucleotide is identical or complementary to at least 18 contiguous nucleotides of an RNA sequence of an ALS gene sequence, wherein said ALS gene sequence is selected from the group consisting of SEQ ID NOs:2, 15, 25-27, 30-33, 35, 36, 39-45, 1692-1697, 1699, 1705, 1707, 1709-1712, 1714-1721, 1727-1742, 1744-1774, and 1776-1788, and whereby said plant treated with said composition has its growth, development, or reproductive ability suppressed or delayed or said plant is more sensitive to an ALS inhibitor herbicide as a result of said composition comprising said dsRNA polynucleotide, relative to an untreated plant.

13. The agricultural chemical composition of claim 12, wherein said pesticide is selected from the group consisting of insecticides, fungicides, nematicides, bactericides, acaricides, growth regulators, chemosterilants, semiochemicals, repellents, attractants, pheromones, feeding stimulants, and biopesticides.

14. A dsRNA polynucleotide for topical application to a surface of a plant, wherein said dsRNA polynucleotide comprises a polynucleotide identical or complementary to at least 18 contiguous nucleotides of an RNA sequence of a polynucleotide selected from the group consisting of SEQ ID NOs:4229-4235, whereby said plant treated with said dsRNA polynucleotide has its sensitivity to a glyphosate containing herbicide composition enhanced, relative to untreated plant.

## Patentansprüche

1. Verfahren zur Kontrolle von Pflanzen, Schritte umfassend, bei denen man eine Zusammensetzung auf eine Oberfläche einer Pflanze aufbringt, die ein doppelsträngiges RNA (dsRNA) Polynukleotid und ein Übertragungsmittel umfasst, wobei das dsRNA-Polynukleotid identisch mit oder komplementär zu mindestens 18 aufeinanderfolgenden Nukleotiden einer RNA-Sequenz einer Acetolactatsynthase (ALS) -Gensequenz ist, wobei die ALS-Gensequenz aus der Gruppe bestehend aus SEQ ID NOs: 2, 15, 25-27, 30-33, 35, 36, 39-45, 1692-1697, 1699, 1705, 1707, 1709-1712, 1714-1721, 1727-1742, 1744-1774 und 1776-1788 ausgewählt wurde, wobei das Übertragungsmittel die Oberfläche der Pflanze für die Durchdringung durch das dsRNA-Polynukleotid konditioniert und wobei das Wachstum, die Entwicklung oder die Fortpflanzungsfähigkeit der Pflanze reduziert ist oder die Pflanze im Vergleich zu einer unbehandelten Pflanze empfindlicher gegenüber einem ALS-Inhibitor-Herbizid ist.

2. Verfahren gemäß Anspruch 1, bei dem
(i) das Übertragungsmittel ist eine siliciumorganische Tensid-Zusammensetzung oder eine darin enthaltene siliciumorganische Verbindung;
(ii) die Pflanze aus der Gruppe bestehend aus *Amaranthus palmeri*, *Amaranthus rudis*, *Amaranthus chlorostachys*, *Amaranthus graecizans*, *Amaranthus hybridus*, *Amaranthus lividus*, *Amaranthus spinosus*, *Amaranthus thunbergii*, *Amaranthus viridis*, *Ambrosia trifida*, *Kochia scopia*, *Abutilon theophrasti*, *Chenopodium album*, *Commelina diffusa*, *Conyza canadensis Digitaria sanguinalis*, *Euphorbia heterophylla* und *Lolium multiflorum* ausgewählt wurde; oder
(iii) die Zusammensetzung ferner folgendes umfasst:
(a) das ALS-Inhibitor-Herbizid; oder
(b) ein oder mehrere Herbizide, die sich von dem ALS-Inhibitor-Herbizid unterscheiden.

3. Verfahren gemäß Anspruch 1, bei dem
das dsRNA-Polynukleotid eine Länge von mindestens 19 aufeinanderfolgenden Nukleotiden hat oder
die Zusammensetzung eine beliebige Kombination aus zwei oder mehr der dsRNA-Polynukleotiden umfasst.

4. Zusammensetzung zur oberflächlichen Anwendung auf einer Oberfläche einer Pflanze, wobei die Zusammensetzung ein dsRNA-Polynukleotid und ein Übertragungsmittel umfasst, wobei das dsRNA-Polynukleotid identisch zu oder komplementär mit mindestens 18 aufeinanderfolgenden Nukleotiden einer RNA-Sequenz einer ALS-Gensequenz ist, wobei die ALS-Gensequenz aus der Gruppe bestehend aus SEQ ID NOs: 2, 15, 25-27, 30-33, 35, 36, 39-45, 1692-1697, 1699, 1705, 1707, 1709-1712, 1714-1721, 1727-1742, 1744-1774 und 1776-1788 ausgewählt wurde, wobei das Übertragungsmittel die Oberfläche der Pflanze für die Durchdringung durch das dsRNA-Polynukleotid konditioniert und wobei die mit der Zusammensetzung behandelte Pflanze ihre Wachstums-, Entwicklungs- oder Reproduktionsfähigkeit unterdrückt oder verzögert oder die Pflanze aufgrund der Zusammensetzung, die das dsRNA-Polynukleotid umfasst, im Vergleich zu einer unbehandelten Pflanze empfindlicher gegenüber einem ALS-Inhibitor-Herbizid ist.

5. Zusammensetzung gemäß Anspruch 4, worin
(i) das Übertragungsmittel eine siliciumorganische Zusammensetzung ist;
(ii) das dsRNA-Polynukleotid mindestens 19 zusammenhängende Nukleotide lang ist;
(iii) das dsRNA-Polynukleotid aus der Gruppe bestehend aus SEQ ID NOs: 48-53, 62, 63, 66-73, 76, 77, 84, 85, 90, 91, 94-97, 100, 101, 110 111, 114-117, 120, 121, 124, 125, 138, 139, 142-145, 148-171, 186-189, 192, 193, 196-239, 250, 251, 254-256, 257, 260 261, 264-279, 288, 289, 294, 295, 306, 307, 310-339, 352-359, 362-365, 378-385, 388-393, 396-419, 428, 429, 432, 433 436, 437, 440, 441, 450, 451, 454-457, 460, 461, 464-467, 476, 477, 480, 481, 486, 487, 490-493, 502, 503, 506-509, 512 513, 516-519, 528, 529, 532-535, 538, 539, 542-559, 564-581, 584-609, 612-631, 634-637, 640-659, 662-665, 668-919 922-1363, 1789-1840, 1843-1916, 1925-2024, 2027-2040, 2043-2060, 2063-2102, 2105-2270, 2273-2286, 2289-2404, 2407-2640, 2647-2662, 2665-2678, 2681-2696, 2699-2956, 2959-3946 und 3949-4166 ausgewählt wurde;
(iv) das dsRNA-Polynukleotid aus der Gruppe bestehend aus SEQ ID NOs: 1364-1691 und 4167-4201 ausgewählt wurde; oder
(v) die Zusammensetzung ferner folgendes umfasst:
(a) das ALS-Inhibitor-Herbizid;
(b) das ALS-Inhibitormolekül, das aus der Gruppe bestehend aus Amidosulfuron, Azimsulfuron, Bensulfuronmethyl, Chlorimuronethyl, Chlorsulfuron, Cinosulfuron, Cyclosulfamuron, Ethamsulfuronmethyl, Ethoxysulfuron, Flazasulfuron, Flupyrsulfuronsulfon, Foramsulfuron, Halosulfuron-methyl, Imazosulfuron, Iodosulfuron, Metsulfuron-Methyl, Nicosulfuron, Oxasulfuron, Primisulfuron-Methyl, Prosulfuron, Pyrazosulfuron-Ethyl, Rimsulfuron, Sulfometuron-Methyl, Sulfosulfuron, Thifensulfuron-methyl, Triasulfuron, Tribenuron-methyl, Trifloxysulfuron, Triflusulfuron-methyl, Tritosulfuron, Imazapic, Imazamethabenz-methyl, Imazamox, Imazapyr, Imazaquin, Imazethapyr, Cloransulammethyl, Diclosulam, Florasulam, Flumetsulam, Metosulam, Bispyribac-Na, Pyribenzoxim, Pyriftalid, Pyrithiobac-Na, Pyriminobac-Methyl, Flucarbazone-Na, and Procarbazone-Na ausgewählt wurde; oder
(c) ein Co-Herbizid.

6. Verfahren zur Verringerung der Expression eines ALS-Gens in einer Pflanze, Schritte umfassend, bei denen man: einer Zusammensetzung, die ein dsRNA-Polynukleotid und ein Übertragungsmittel umfasst, auf eine Oberfläche der Pflanze aufbringt, wobei das dsRNA-Polynukleotid identisch zu oder komplementär mit mindestens 18 aufeinanderfolgenden Nukleotiden einer RNA Sequenz einer ALS-Gensequenz ist, wobei die ALS-Gensequenz aus der Gruppe bestehend aus SEQ ID NOs: 2, 15, 25-27, 30-33, 35, 36, 39-45, 1692-1697, 1699, 1705 1707, 1709-1712, 1714-1721, 1727-1742, 1744-1774 und 1776-1788 ausgewählt wurde, wobei das Übertragungsmittel die Oberfläche der Pflanze für die Durchdringung durch das dsRNA-Polynukleotid konditioniert, wodurch die Expression des ALS-Gens im Vergleich zu einer unbehandelten Pflanze verringert wird.

7. Verfahren nach Anspruch 6, wobei
(i) das Übertragungsmittel eine siliciumorganische Zusammensetzung ist; oder
(ii) das dsRNA-Polynukleotid hat eine Länge von mindestens 19 zusammenhängenden Nukleotiden.

8. Verfahren zur Identifizierung von dsRNA-Polynukleotiden, die zur Veränderung der ALS-Genexpression verwendet werden können, wenn sie auf eine Oberfläche einer Pflanze aufgebracht werden, Schritte umfassend, bei denen man: a) eine Vielzahl an dsRNA-Polynukleotiden bereitstellt, die eine Region umfassen, in der mindestens 18 aufeinanderfolgenden Nukleotiden der RNA-Sequenz identisch zu oder komplementär mit einer ALS-Gensequenz sind, die aus der Gruppe bestehend aus SEQ ID NOs: 2, 15, 25-27, 30-33, 35, 36, 39-45, 1692-1697, 1699, 1705, 1707, 1709-1712, 1714-1721 1727-1742, 1744-1774 und 1776-1788 ausgewählt wurde; b) eine Zusammensetzung auf die Oberfläche der Pflanze aufbringt, die ein oder mehrere der dsRNA-Polynukleotide und ein Übertragungsmittel umfasst; und c) die Pflanze oder einen Pflanzenextrakts davon auf eine Veränderung der ALS-Genexpression hin analisiert, wobei das Übertragungsmittel die Oberfläche der Pflanze für eine Durchdringung von einer oder mehrerer der dsRNA-Polynukleotide konditioniert und wobei das Wachstum, die Entwicklung oder die Fortpflanzungsfähigkeit der Pflanze, die mit der Zusammensetzung behandelt wurde, unterdrückt oder verzögert wird, oder die Pflanze aufgrund der Zusammensetzung im Vergleich zu einer unbehandelten Pflanze empfindlicher gegenüber einem ALS-Inhibitor-Herbizid ist.

9. Verfahren nach Anspruch 8, wobei
(i) die Pflanze aus der Gruppe bestehend aus *Amaranthus palmeri*, *Amaranthus rudis*, *Amaranthus chlorostachys*, *Amaranthus graecizans*, *Amaranthus hybridus*, *Amaranthus lividus*, *Amaranthus spinosus*, *Amaranthus thunbergii*, *Amaranthus viridis*, *Ambrosia trifida*, *Kochia scopia*, *Abutilon theophrasti*, *Chenopodium album*, *Commelina diffusa*, *Conyza canadensis Digitaria sanguinalis*, *Euphorbia heterophylla* und *Lolium multiflorum* ausgewählt wurde; oder
(ii) die ALS-Genexpression im Vergleich zu einer Pflanze, die nicht mit der Zusammensetzung behandelt wurde, verringert ist; oder
(iii) das Übertragungsmittel eine siliciumorganische Zusammensetzung ist.

10. Agrarchemische Zusammensetzung zur oberflächlichen Anwendung auf eine Oberfläche einer Pflanze, die ein Gemisch aus einem dsRNA-Polynukleotid, Glyphosat und einem Co-Herbizid umfasst, wobei das dsRNA-Polynukleotid identisch zu oder komplementär mit mindestens 18 aufeinanderfolgenden Nukleotiden einer RNA-Sequenz einer ALS-Gensequenz ist, wobei die ALS-Gensequenz aus der Gruppe bestehend aus SEQ ID NOs: 2, 15, 25-27, 30-33, 35, 36, 39-45, 1692-1697, 1699, 1705, 1707, 1709 -1712, 1714-1721, 1727-1742, 1744-1774 und 1776-1788 ausgewählt wurde, wobei bei der mit der Zusammensetzung behandelten Pflanze das Wachstum, die Entwicklung oder die Fortpflanzungsfähigkeit unterdrückt oder verzögert wird oder die Pflanze aufgrund der Zusammensetzung, die das dsRNA-Polynukleotid umfasst, empfindlicher als eine unbehandelte Pflanze auf ein ALS-Inhibitor-Herbizid reagiert.

11. Landwirtschaftliche chemische Zusammensetzung gemäß Anspruch 10, in der das Co-Herbizid aus der Gruppe bestehend aus Amid-Herbiziden, Arsen-haltigen Herbiziden, Benzothiazol Herbizide, Benzoylcyclohexandione Herbizid, Benzofuranyl-Alkylsulfonat Herbiziden, Carbamat-Herbiziden, Cyclohexen-Oxim Herbiziden, cyclopropylisoxazol Herbiziden, Dicarboximid-Herbiziden, Dinitroanilin Herbiziden, Dinitrophenol-Herbiziden, Diphenylether-Herbiziden, Dithiocarbamat-Herbiziden, halogenierten aliphatischen Herbiziden, Imidazolinon-Herbiziden, anorganischen Herbiziden, Nitril-Herbiziden, organischen Phosphor-Herbiziden, Oxadiazolon-Herbiziden, Oxazol-Herbiziden, Phenoxy-Herbiziden, Phenylendiamin-Herbiziden, Pyrazol-Herbiziden, Pyridazin-Herbiziden, Pyridazinon-Herbiziden, Pyridin-Herbiziden, Pyrimidindiamin-Herbiziden, Pyrimidinyloxybenzylamin-Herbiziden, quaternäre Ammoniumherbiziden, Thiocarbamat-Herbiziden, Thiocarbonat-Herbiziden, Thioharnstoff-Herbiziden, Triazin-Herbiziden, Triazinon Herbiziden, Triazolherbiziden, Triazolonherbiziden, Triazolopyrimidin Herbiziden, Uracil Herbiziden und Harnstoff Herbiziden ausgewählt wurde.

12. Landwirtschaftliche chemische Zusammensetzung zur oberflächlichen Anwendung auf einer Oberfläche einer Pflanze, umfassend ein Gemisch aus einem dsRNA-Polynukleotid, Glyphosat und einem Pestizid, wobei das dsRNA-Polynukleotid identisch zu oder komplementär mit mindestens 18 aufeinanderfolgenden Nukleotiden einer RNA-Sequenz einer ALS-Gensequenz ist, wobei die ALS-Gensequenz aus der Gruppe bestehend aus SEQ ID NOs: 2, 15, 25-27, 30-33, 35, 36, 39-45, 1692-1697, 1699, 1705, 1707, 1709-1712 1714-1721, 1727-1742, 1744-1774 und 1776-1788 ausgewählt wurde, und wobei das Wachstum, die Entwicklung oder die Fortpflanzungsfähigkeit der mit der Zusammensetzung behandelten Pflanze unterdrückt oder verzögert ist oder die Pflanze aufgrund der Zusammensetzung, die das dsRNA-Polynukleotid umfasst, im Vergleich zu einer unbehandelten Pflanze empfindlicher auf ein ALS-Inhibitor-Herbizid reagiert.

13. Landwirtschaftliche chemische Zusammensetzung gemäß Anspruch 12, wobei das Pestizid aus der Gruppe bestehend aus Insektiziden, Fungiziden, Nematiziden, Bakteriziden, Akariziden, Wachstumsregulatoren, Chemosterilantien, Semiochemikalien, Repellentien, Lockstoffen, Pheromonen, Fütterungsstimulanzien und Biopestiziden ausgewählt wurde.

14. dsRNA-Polynukleotid zur oberflächlichen Anwendung auf einer Oberfläche einer Pflanze, wobei das dsRNA-Polynukleotid ein Polynukleotid umfasst, das identisch mit oder komplementär zu mindestens 18 aufeinanderfolgendne Nukleotiden einer RNA-Sequenz eines Polynukleotids ist, das aus der Gruppe bestehend aus SEQ ID NOs: 4229-4235 ausgewählt wurde, wobei eine mit dem dsRNA-Polynukleotid behandelte Pflanze im Vergleich zu einer unbehandelten Pflanze eine empfindlicher auf eine Glyphosat-enthaltende Herbizid-Zusammensetzung reagiert.

## Revendications

1. Procédé de lutte contre des plantes, comprenant : le fait d'appliquer topiquement sur une surface d'une plante une composition comprenant un polynucléotide ARN double brin (ARNdb) et un agent de transfert, dans lequel ledit polynucléotide ARNdb est identique à ou complémentaire d'au moins 18 nucléotides contigus d'une séquence d'ARN d'une séquence de gène d'acétolactate synthase (ALS), dans lequel ladite séquence de gène d'ALS est choisie dans l'ensemble constitué par les séquences SEQ ID NO: 2, 15, 25-27, 30-33, 35, 36, 39-45, 1692-1697, 1699, 1705, 1707, 1709-1712, 1714-1721, 1727-1742, 1744-1774, et 1776-1788, dans lequel ledit agent de transfert conditionne ladite surface de ladite plante pour la perméation par ledit polynucléotide ARNdb, et ce par quoi la croissance, le développement, ou l'aptitude à la reproduction de ladite plante est réduit(e) ou ladite plante est plus sensible à un herbicide inhibiteur d'ALS, par rapport à une plante non traitée.

2. Procédé selon la revendication 1, dans lequel
(i) ledit agent de transfert est une composition de tensioactif organosilicone ou un composé organosilicone contenu dans celle-ci ;
(ii) ladite plante est choisie dans l'ensemble constitué par *Amaranthus palmeri, Amaranthus rudis, Amaranthus chlorostachys*, *Amaranthus graecizans, Amaranthus hybridus, Amaranthus lividus, Amaranthus spinosus, Amaranthus thunbergii, Amaranthus viridis, Ambrosia trifida, Kochia scoparia, Abutilon theophrasti*, *Chenopodium album, Commelina diffusa*, *Conyza canadensis Digitaria sanguinalis, Euphorbia heterophylla*, et *Lolium multiflorum* ; ou
(iii) ladite composition comprend en outre
(a) ledit herbicide inhibiteur d'ALS ; ou
(b) un ou plusieurs herbicide(s) différent(s) dudit herbicide inhibiteur d'ALS.

3. Procédé selon la revendication 1, dans lequel
ledit polynucléotide ARNdb a une longueur d'au moins 19 nucléotides contigus, ou
ladite composition comprend une association quelconque de deux ou plus de deux desdits polynucléotides ARNdb.

4. Composition destinée à l'application topique sur une surface d'une plante, comprenant un polynucléotide ARNdb et un agent de transfert, dans laquelle ledit polynucléotide ARNdb est identique à ou complémentaire d'au moins 18 nucléotides contigus d'une séquence d'ARN d'une séquence de gène d'ALS, dans laquelle ladite séquence de gène d'ALS est choisie dans l'ensemble constitué par les séquences SEQ ID NO: 2, 15, 25-27, 30-33, 35, 36, 39-45, 1692-1697, 1699, 1705, 1707, 1709-1712, 1714-1721, 1727-1742, 1744-1774, et 1776-1788, dans laquelle ledit agent de transfert conditionne ladite surface de ladite plante pour la perméation par ledit polynucléotide ARNdb, et ce par quoi ladite plante traitée par ladite composition présente une suppression ou un retard de sa croissance, de son développement, ou de son aptitude à la reproduction ou ladite plante est plus sensible à un herbicide inhibiteur d'ALS, par suite du fait que ladite composition comprend ledit polynucléotide ARNdb, par rapport à une plante non traitée.

5. Composition selon la revendication 4, dans laquelle
(i) ledit agent de transfert est une préparation d'organosilicone ;
(ii) ledit polynucléotide ARNdb a une longueur d'au moins 19 nucléotides contigus ;
(iii) ledit polynucléotide ARNdb est choisi dans l'ensemble constitué par les séquences SEQ ID NO: 48-53, 62, 63, 66-73, 76, 77, 84, 85, 90, 91, 94-97, 100, 101, 110, 111, 114-117, 120, 121, 124, 125, 138, 139, 142-145, 148-171, 186-189, 192, 193, 196-239, 250, 251, 254-256, 257, 260, 261, 264-279, 288, 289, 294, 295, 306, 307, 310-339, 352-359, 362-365, 378-385, 388-393, 396-419, 428, 429, 432, 433, 436, 437, 440, 441, 450, 451, 454-457, 460, 461., 464-467, 476, 477, 480, 481, 486, 487, 490-493, 502, 503, 506-509, 512, 513, 516-519, 528, 529, 532-535, 538, 539, 542-559, 564-581, 584-609, 612-631, 634-637, 640-659, 662-665, 668-919, 922-1363, 1789-1840, 1843-1916, 1925-2024, 2027-2040, 2043-2060, 2063-2102, 2105-2270, 2273-2286, 2289-2404, 2407-2640, 2647-2662, 2665-2678, 2681-2696, 2699-2956, 2959-3946, et 3949-4166 ;
(iv) ledit polynucléotide ARNdb est choisi dans l'ensemble constitué par les séquences SEQ ID NO: 1364-1691 et 4167-4201 ; ou
(v) ladite composition comprend en outre
(a) ledit herbicide inhibiteur d'ALS ;
(b) ladite molécule d'inhibiteur d'ALS choisie dans l'ensemble constitué par l'amidosulfuron, l'azimsulfuron, le bensulfuron-méthyle, le chlorimuron-éthyle, le chlorsulfuron, le cinosulfuron, le cyclosulfamuron, l'éthametsulfuron-méthyle, l'éthoxysulfuron, le flazasulfuron, le flupyrsulfuron-méthyl-Na, le foramsulfuron, l'halosulfuron-méthyle, l'imazosulfuron, l'iodosulfuron, le metsulfuron-méthyle, le nicosulfuron, l'oxasulfuron, le primisulfuron-méthyle, le prosulfuron, le pyrazosulfuron-éthyle, le rimsulfuron, le sulfométuron-méthyle, le sulfosulfuron, le thifensulfuron-méthyle, le triasulfuron, le tribénuron-méthyle, le trifloxysulfuron, le triflusulfuron-méthyle, le tritosulfuron, l'imazapic, l'imazaméthabenz-méthyle, l'imazamox, l'imazapyr, l'imazaquine, l'imazéthapyr, le cloransulame-méthyle, le diclosulame, le florasulame, le flumetsulame, le métosulame, le bispyribac-Na, le pyribenzoxime, le pyriftalide, le pyrithiobac-Na, le pyriminobac-méthyle, la flucarbazone-Na, et la procarbazone-Na ; ou
(c) un co-herbicide.

6. Procédé de réduction de l'expression d'un gène d'ALS chez une plante, comprenant : le fait d'appliquer topiquement sur une surface de ladite plante une composition comprenant un polynucléotide ARNdb et un agent de transfert, dans lequel ledit polynucléotide ARNdb est identique à ou complémentaire d'au moins 18 nucléotides contigus d'une séquence d'ARN d'une séquence de gène d'ALS, dans lequel ladite séquence de gène d'ALS est choisie dans l'ensemble constitué par les séquences SEQ ID NO: 2, 15, 25-27, 30-33, 35, 36, 39-45, 1692-1697, 1699, 1705, 1707, 1709-1712, 1714-1721, 1727-1742, 1744-1774, et 1776-1788, dans lequel le ledit agent de transfert conditionne ladite surface de ladite plante pour la perméation par ledit polynucléotide ARNdb, ce par quoi ladite expression dudit gène d'ALS est réduite, par rapport à une plante non traitée.

7. Procédé selon la revendication 6, dans lequel
(i) ledit agent de transfert est une préparation d'organosilicone ; ou
(ii) ledit polynucléotide ARNdb a une longueur d'au moins 19 nucléotides contigus.

8. Procédé d'identification de polynucléotides ARNdb utiles dans la modulation de l'expression d'un gène d'ALS lorsqu'ils sont appliqués topiquement sur une surface d'une plante, comprenant les étapes consistant à : a) fournir une pluralité de polynucléotides ARNdb qui comprennent une région identique à ou complémentaire d'au moins 18 nucléotides contigus d'une séquence d'ARN d'une séquence de gène d'ALS, choisie dans l'ensemble constitué par les séquences SEQ ID NO: 2, 15, 25-27, 30-33, 35, 36, 39-45, 1692-1697, 1699, 1705, 1707, 1709-1712, 1714-1721, 1727-1742, 1744-1774, et 1776-1788 ; b) appliquer topiquement sur ladite surface de ladite plante une composition comprenant un ou plusieurs desdits polynucléotides ARNdb et un agent de transfert ; et c) analyser ladite plante, ou un extrait végétal de celle-ci, pour mettre en évidence une modulation de l'expression de gène d'ALS, dans lequel ledit agent de transfert conditionne ladite surface de ladite plante pour la perméation par le(s)dits(s) un ou plusieurs desdits polynucléotides ARNdb, et ce par quoi ladite plante traitée par ladite composition présente une suppression ou un retard de sa croissance, de son développement, ou de son aptitude à la reproduction ou ladite plante est plus sensible à un herbicide inhibiteur d'ALS, par suite de ladite composition, par rapport à une plante non traitée.

9. Procédé selon la revendication 8, dans lequel
(i) ladite plante est choisie dans l'ensemble constitué par *Amaranthus palmeri, Amaranthus rudis, Amaranthus chlorostachys*, *Amaranthus graecizans, Amaranthus hybridus, Amaranthus lividus, Amaranthus spinosus, Amaranthus thunbergii, Amaranthus viridis, Ambrosia trifida, Kochia scoparia*, *Abutilon theophrasti*, *Chenopodium album, Commelina diffusa, Conyza canadensis Digitaria sanguinalis, Euphorbia heterophylla*, et *Lolium multiflorum* ; or
(ii) ladite expression de gène d'ALS est réduite par rapport à une plante non traitée par ladite composition ; ou
(iii) ledit agent de transfert est une préparation d'organosilicone.

10. Composition chimique agricole destinée à l'application topique sur une surface d'une plante, comprenant un mélange d'un polynucléotide ARNdb, de glyphosate, et d'un co-herbicide, dans laquelle ledit polynucléotide ARNdb est identique à ou complémentaire d'au moins 18 nucléotides contigus d'une séquence d'ARN d'une séquence de gène d'ALS, dans laquelle ladite séquence de gène d'ALS est choisie dans l'ensemble constitué par les séquences SEQ ID NO: 2, 15, 25-27, 30-33, 35, 36, 39-45, 1692-1697, 1699, 1705, 1707, 1709-1712, 1714-1721, 1727-1742, 1744-1774, et 1776-1788, et ce par quoi ladite plante traitée par ladite composition présente une suppression ou un retard de sa croissance, de son développement, ou de son aptitude à la reproduction ou ladite plante est plus sensible à un herbicide inhibiteur d'ALS, par suite du fait que ladite composition comprend ledit polynucléotide ARNdb, par rapport à une plante non traitée.

11. Composition chimique agricole selon la revendication 10, dans laquelle ledit co-herbicide est choisi dans l'ensemble constitué par les herbicides de type amide, les herbicides arsénicaux, les herbicides de type benzothiazole, les herbicides de type benzoylcyclohexanedione, les herbicides de type alkylsulfonate de benzofuranyle, les herbicides de type carbamate, les herbicides de type cyclohexène-oxime, les herbicides de type cyclopropylisoxazole, les herbicides de type dicarboximide, les herbicides de type dinitroaniline, les herbicides de type dinitrophénol, les herbicides de type éther diphénylique, les herbicides de type dithiocarbamate, les herbicides aliphatiques halogénés, les herbicides de type imidazolinone, les herbicides inorganiques, les herbicides de type nitrile, les herbicides organophosphorés, les herbicides de type oxadiazolone, les herbicides de type oxazole, les herbicides de type phénoxy, les herbicides de type phénylènediamine, les herbicides de type pyrazole, les herbicides de type pyridazine, les herbicides de type pyridazinone, les herbicides de type pyridine, les herbicides de type pyrimidinediamine, les herbicides de type pyrimidinyloxybenzylamine, les herbicides de type ammonium quaternaire, les herbicides de type thiocarbamate, les herbicides de type thiocarbonate, les herbicides dérivés de thiourée, les herbicides de type triazine, les herbicides de type triazinone, les herbicides de type triazole, les herbicides de type triazolone, les herbicides de type triazolopyrimidine, les herbicides à base d'uracile et les herbicides dérivés d'urée.

12. Composition chimique agricole destinée à l'application topique sur une surface d'une plante, comprenant un mélange d'un polynucléotide ARNdb, de glyphosate, et d'un pesticide, dans laquelle ledit polynucléotide ARNdb est identique à ou complémentaire d'au moins 18 nucléotides contigus d'une séquence d'ARN d'une séquence de gène d'ALS, dans laquelle ladite séquence de gène d'ALS est choisie dans l'ensemble constitué par les séquences SEQ ID NO: 2, 15, 25-27, 30-33, 35, 36, 39-45, 1692-1697, 1699, 1705, 1707, 1709-1712, 1714-1721, 1727-1742, 1744-1774, et 1776-1788, et ce par quoi ladite plante traitée par ladite composition présente une suppression ou un retard de sa croissance, de son développement, ou de son aptitude à la reproduction ou ladite plante est plus sensible à un herbicide inhibiteur d'ALS, par suite du fait que ladite composition comprend ledit polynucléotide ARNdb, par rapport à une plante non traitée.

13. Composition chimique agricole selon la revendication 12, dans laquelle ledit pesticide est choisi dans l'ensemble constitué par les insecticides, fongicides, nématicides, bactéricides, acaricides, régulateurs de croissance, chimiostérilisants, substances sémiochimiques, répulsifs, attractants, phéromones, phagostimulants et biopesticides.

14. Polynucléotide ARNdb destiné à l'application topique sur une surface d'une plante, où ledit polynucléotide ARNdb comprend un polynucléotide identique à ou complémentaire d'au moins 18 nucléotides contigus d'une séquence d'ARN d'un polynucléotide choisi dans l'ensemble constitué par les séquences SEQ ID NO: 4229-4235, ce par quoi ladite plante traitée par ledit polynucléotide ARNdb présente une augmentation de sa sensibilité à une composition d'herbicide contenant du glyphosate, par rapport à une plante non traitée.
